# EUROPÄISCHE PATENTSCHRIFT

(11) **EP 1 796 491 B1**
(45) Veröffentlichungstag und Bekanntmachung des Hinweises auf die Patenterteilung: **19.11.2008**
(21) Anmeldenummer: 05784438.3
(22) Anmeldetag: 26.09.2005
(51) Int. Cl.: A41D 19/00, A41D 31/02, A41D 27/24

(54) **INNENAUSKLEIDUNG UND VERFAHREN ZU DEREN HERSTELLUNG**
INTERNAL LINING AND METHOD FOR THE PRODUCTION THEREOF
REVETEMENT INTERIEUR ET SON PROCEDE DE FABRICATION

(30) Priorität: 07.10.2004 AT 16762004
(43) Veröffentlichungstag der Anmeldung: 20.06.2007
(73) Patentinhaber: Eska Lederhandschuhfabrik Gesellschaft m.b.H. & Co. KG, 4600 Thalheim bei Wels (AT)
(72) Erfinder: LOOS, Paul, A-4600 Thalheim bei Wels (AT)
(74) Vertreter: Secklehner, Günter
(86) Internationale Anmeldenummer: PCT/AT2005/000385
(87) Internationale Veröffentlichungsnummer: WO 2006/037138

(56) Entgegenhaltungen:
- EP-A- 0 328 421
- US-A- 5 442 818
- US-A- 5 631 074
- US-A1- 2001 008 672
- US-A1- 2003 131 635
- PATENT ABSTRACTS OF JAPAN Bd. 014, Nr. 458 (C-0766), 3. Oktober 1990 (1990-10-03) & JP 02 182902 A (KURARAY CO LTD), 17. Juli 1990 (1990-07-17)

## Beschreibung

Die Erfindung betrifft eine Innenauskleidung für einen Ausrüstungsgegenstand, zum zumindest temporären Bedecken von Körperteilen, insbesondere Kleidungsstücke, Decken, Schlafsäcke, aus mehreren Schichten, wobei zumindest eine Schicht aus einem Gewebe und/oder Gewirke bzw. aus Leder angeordnet ist, ein Verfahren zur Herstellung einer Innenauskleidung, mit welchem mehrere Schichten, insbesondere zumindest eine Schicht aus einem Gewebe und/oder Gewirke bzw. Leder mit antimikrobieller Wirkung und einer weiteren Schicht aus semipermeabler Membran der gewünschten Innenauskleidung entsprechend in einzelne Schnittteile zugeschnitten und geformt werden oder alternativ bereits verbunden zugeschnitten und geformt werden, worauf die zumindest zwei Schichten zumindest partiell deckend und gegebenenfalls semipermeabel miteinander verbunden werden, die Verwendung der Innenauskleidung und einen Ausrüstungsgegenstand mit einer Innenauskleidung.

Innenauskleidungen für Ausrüstungsgegenstände werden in vielfältiger Art und Weise für die unterschiedlichsten Anwendungsfälle eingesetzt.

So müssen Innenauskleidungen desselben Ausrüstungsgegenstandes oft unterschiedlichsten Anforderungen entsprechen. Beispielsweise müssen sowohl wärmeisolierende als auch wasserabweisende aber dennoch luftdurchlässige Eigenschaften in einem Gegenstand vereint sein. Zudem steigt in der heutigen Zeit ständig die Nachfrage der Verbraucher nach funktioneller Bekleidung. Wobei neben der wasserabweisenden aber schweißdurchlässigen Kleidung auch immer mehr die Nachfrage nach antimikrobiell behandelter Kleidung, aufgrund des wachsenden Hygienebewusstseins und des Wissens um die potentiell schädlichen Auswirkungen von Mikroorganismen ansteigt.

Aufgrund der unterschiedlichen Eigenschaften von Membranen und Gewebe bzw. Gewirke ist sowohl deren gemeinsame Verarbeitung problematisch, als auch der Tragekomfort von damit ausgestatteten Kleidungsstücken nicht immer optimal.

Beispielsweise werden einzelne Schnittteile für Handschuh-Futter sowohl für die Membran als auch für den textilen Futterstoff zugeschnitten und müssen zusammengefügt werden. Da beim Zusammennähen der Membran-Schnittteile die Handschuh-Innenauskleidung an den Nahtstichen durchlässig werden würde, müssen diese verklebt oder verschweißt werden, oder es müssen wenigstens nach allfälligem Zusammennähen die Nähte mit einem dichtenden Mittel überdeckt werden. Die Schnittteile aus dem textilen Futterstoffhingegen werden in üblicher Weise zusammengenäht. Die beiden so erhaltenen Futterschichten aus textilem Stoff und Membran werden sodann gemeinsam an das Handschuh-Obermaterial angenäht. Das der Hand zugekehrte, wärmedämmende, textile Stoff-Futter, das dazu tendiert, an der immer etwas feuchten Hand zu haften bzw. einen unangenehmen Geruch zu entwickeln, wird beim Ausziehen des Handschuhs unter gleichzeitigem Umstülpen leicht aus dem Handschuh herausgezogen, insbesondere, da die zwischen Stoff-Futter und Handschuh-Obermaterial liegende Membran glatte und, aufgrund ihrer Wasser abstoßenden Eigenschaft, trockene Oberflächen hat.

Die US 2003/0131635 A1 beschreibt einen gestrickten Socken mit einer Innen- und einer Außenschicht, die einen gemeinsamen Zehenteil formen. Der Fußbereich der Innenschicht wird mit einem Garn hergestellt, welches einen niedrigen Reibungskoeffizienten und/oder fungizide bzw. antibakterielle Eigenschaften aufweist. Die Faser mit geringem Reibungskoeffizienten ist ein Fluorpolymer, wie z.B. Polytetrafluorethylen. Zumindest ein Teil der äußeren Schicht des Sockens umfasst ein technisches Garn, welches eine Faser mit fungiziden bzw. antibakteriellen Eigenschaften sein kann. Die Faser ist z.B. mit Silber beschichtetes Nylon. Somit beschreibt die US-A1 einen zweischichtigen gestrickten Socken, der aus einer Innenschicht und einer Außenschicht besteht. Die Innenschicht wird aus einem Garn gestrickt, welches als erste Faser Polytetrafluorethylen enthält. Die äußere Schicht umfasst eine technische Faser, die fungizide bzw. antibakterielle Eigenschaften aufweist. Die antimikrobiellen Eigenschaften werden durch eine Beschichtung des Nylons mit Silber hervorgerufen.

In der WO 89/07523 wird dargestellt, wie - insbesondere semipermeable - Membranen mit dehnbarem, - insbesondere gestricktem oder gewirktem - textilem Material ein- oder auch zweiseitig zu verbinden ist, um beispielsweise Innenfutter für Handschuhe und Socken, bzw. Handschuhe und Socken selbst, herzustellen. Derartige Handschuhe oder Socken sollen sich durch besonderen Tragekomfort - in Bezug auf Wasserabstoßung und Wärmeisolation - auszeichnen, was einerseits auf den vollständigen Verzicht von Perforationen der Membran und andererseits auf die kleinste Luftkammern bildende Kräuselung bei der Herstellung zurückzuführen ist. Allerdings ist die Herstellung solcher Handschuhe oder Socken bzw. deren Innenfutter auf die Verwendung von fertig gewirkten bzw. gestrickten Handschuh- bzw.

Socken-Textilschichten beschränkt, die gedehnt und mit Membranschicht-Teilen belegt und großflächig partiell verklebt werden, wobei die Membranschicht-Teile an ihren Überlappungen miteinander verschweißt werden. Eine weitere textile Schicht kann auf die andere Seite der Membranschicht nur über nochmaliges Dehnen der Membran-Textilschicht und der weiteren Textilschicht und durch analoges Verkleben aufgebracht bzw. in oben dargestellter Weise über schlaufenähnliche Ansätze befestigt werden.

Kleidungsstücke, für die andere, als dehnbare, d.h. insbesondere gewirkte oder gestrickte, textile Stoffmaterialen verwendet werden sollen, können auf diese in der WO 89/07523 beschriebene Art und Weise nicht mit einem eine semipermeable Membran enthaltenden Innenfutter versehen werden. Ebenso wenig eignet sich dieser Herstellungsart für schneidertechnisch komplizierte Kleidungsstücke, wobei auf gute Passform, die den Konfektionsgrößen entsprechen, Wert gelegt wird. Dass ein Kleidungsstück, bzw. dessen Innenfutter, das auf diese Art hergestellt wurde, d.h. Dehnen - (Heiß-)Verkleben - Zusammenschrumpfen und gegebenenfalls neuerlichem Dehnen - (Heiß-)Verkleben - Zusammenschrumpfen, nur begrenzt diese Forderung erfüllen wird, ist zu erwarten.

Aus der JP 06136603 A ist die Herstellung von Handschuhen mit antibakterieller und deodorisierender Funktion bekannt. Um eine effiziente antibakterielle und deodorisierende Eigenschaft des Handschuhs zu bewirken, wird auf die Innenseite der gewebten Faser eine entsprechende Behandlung angewendet. Es wird ein Puder einer antibakteriell metallischen Zusammensetzung in Kombination mit einem thermoplastischen synthetischen Harz in Wasser oder in einem Lösungsmittel aufgebracht, welches einen niedrigen Schmelzpunkt aufweist. Die Suspension adsorbiert an die Innenseite der Gewebestruktur des Handschuhs und wird durch Wärmebehandlung mit einer Temperatur oberhalb des Erweichungspunkt des niedrig schmelzenden thermoplastischen synthetischen Harzes getrocknet. Somit kann die Entstehung von üblem Geruch in Handschuhen unterdrückt werden, insbesondere beim Hantieren von rohem Fisch, wobei der Arbeitshandschuh zudem eine niedrige Luftpermeabilität aufweist.

Aus der JP 2001/299982 A ist ein Sporthandschuh bekannt, welcher aus Leder besteht und für die Verwendung bei Golf oder anderen Sportarten eingesetzt werden kann. Die Aufgabenstellung der JP-A besteht darin, dass der Handschuh auch bei Feuchtigkeit gute Rutscheigenschaften und somit einen hohen Anziehkomfort aufweist. Die Aufgabenstellung wird dadurch gelöst, dass ein Handschuh aus Naturleder zur Verfügung gestellt wird, von welchem eine Oberflächenlage aus Silberoberfläche abgezogen wird. Bei diesem Sporthandschuh wird die Silberoberflächenseite zur Innenseite des Handschuhs gemacht.

Aufgabe der Erfindung ist es, eine Innenauskleidung für einen Ausrüstungsgegenstand zur Verfügung zu stellen, welche einen Tragekomfort bietet und mit welcher eine unangenehme Geruchsbildung mit effizienter Wirkung und gleichzeitig ohne Sicherheitsrisiko für den Verbraucher oder die Umwelt verhindert werden kann.

Die Aufgabe der Erfindung wird dadurch gelöst, dass eine Innenauskleidung zur Verfügung gestellt wird, welche zumindest eine Schicht aus einem Gewebe und/oder Gewirke bzw. Leder mit antimikrobieller Wirkung aufweist, ein Verfahren zur Herstellung einer Innenauskleidung, mit welchen mehrere Schichten, insbesondere zumindest eine Schicht aus einem Gewebe und/oder Gewirke bzw. Leder mit antimikrobieller Wirkung und eine weitere Schicht aus semipermeabler Membran der gewünschten Innenauskleidung entsprechend in einzelne Schnittteile zugeschnitten und geformt werden, worauf zumindest diese zwei Schichten zumindest partiell deckend und gegebenenfalls semipermeabel miteinander verbunden werden, ein Verfahren zur Herstellung einer Innenauskleidung, wobei die zumindest eine Schicht aus einem Gewebe und/oder Gewirke bzw. Leder mit antimikrobieller Wirkung und die zumindest eine Schicht aus semipermeabler Membran vor dem Zuschneiden zumindest partiell miteinander verbunden werden, worauf diese Schichten gemeinsam zugeschnitten und die Schnittteile der Innenauskleidungsform entsprechend zusammengefügt werden, die Verwendung der Innenauskleidung und einen Ausrüstungsgegenstand mit einer Innenauskleidung, wobei dieser aus einer Schicht aus semipermeabler Membran und einer Schicht aus Gewebe und/oder Gewirke bzw. Leder mit antimikrobiellen Eigenschaften und gegebenenfalls einer zusätzlichen Schicht aus einem Gewebe und/oder Gewirke bzw. Leder gebildet ist. Der Einsatz von antimikrobiell funktionalisierter Bekleidung ermöglicht, dass sich die Produkte auf dem Markt von anderen unterscheiden, weil sie den Aspekt der Sauberkeit und der Frische der antimikrobiell behandelten Bekleidungsstücke hervorheben. Es sind sowohl die Forderungen des Verbrauchers nach Komfort als auch nach Funktionalität erfüllt. Vorteilhaft dabei erweist sich, dass durch die antimikrobielle Wirkung das Wachstum von Bakterien, Viren, Pilzen und anderen Mikroorganismen verhindert werden kann und dadurch bei der Verwendung der Innenauskleidung zur Herstellung von Ausrüstungsgegenständen die unangenehme Geruchsbildung bzw. das psychologische Unbehangen in Zusammenhang mit geruchsbildenden Bakterien und die Entstehung von durch Pilze verursachte Hauterkrankungen vermieden werden kann. Zudem erweist sich dabei von Vorteil, dass durch die antimikrobiellen Eigenschaften der Innenauskleidung diese auch beispielsweise auf verletztes Gewebe bzw. Wunden aufgelegt werden kann ohne Infektionen zu verursachen bzw. durch Durchblutungssteigerung sogar den Wundheilungsprozess zu fördern. Von Vorteil ist auch, dass durch diese Schicht mit antimikrobieller Wirkung eine starke Barriere gegen die Ausbreitung von Bakterien, die resistent gegen Antibiotika und die für die in Krankenhäusern auftretenden Infektionen verantwortlich sind, geboten wird. In vielen industrialisierten Ländern steigt nämlich die Zahl solcher Infektionen.

Durch die Weiterbildung der Erfindung durch die Anordnung zumindest einer weiteren Schicht aus einer semipermeablen Membran, insbesondere aus Polytetrafluorethylen (PTFE), einem Laminat, gebildet aus Polyvinylchlorid, Polyurethan, Polyetheramid, Polyetheresteramid, Polyolefinkomponenten, Polyesterkomponenten oder einem Gemisch daraus, wird ermöglicht, dass die Innenauskleidung auch wasserabweisend, aber dennoch luftdurchlässig ist. Von Vorteil dabei erweist sich, dass die Temperatur reguliert werden kann und dadurch die Transpiration und somit der Abtransport von Flüssigkeit von der Haut des Trägers auf die Oberfläche des Ausrüstungsgegenstands ermöglicht wird.

In einer Weiterbildung ist vorgesehen, dass das Gewebe und/oder Gewirke bzw. Leder die antimikrobielle Wirkung durch ein Metall erhält, wobei das Metall aus einer Gruppe umfassend Silber, Gold, Kupfer, Platinium und/oder einem Gemisch daraus ausgewählt ist, wodurch lediglich Elemente verwendet werden, die natürlich sind. Deshalb kommen keine Chemikalien zum Einsatz. Das Tragen einer Innenauskleidung mit solchen Elementen ist sowohl für den Träger sehr angenehm, als auch für die Umwelt absolut unschädlich.

Es ist vorgesehen, dass das Metall, insbesondere Silber, in einem Prozentanteil mit einer oberen Grenze von 80%, vorzugsweise 35%, insbesondere 20% und einer unteren Grenze von 0,5%, vorzugsweise 1%, insbesondere 5% in der Schicht aus einem Gewebe und/oder Gewirke bzw. Leder mit antimikrobieller Wirkung enthalten ist, wodurch die ursprünglichen angenehmen Trageeigenschaften bzw. der Tragekomfort und die Tastgeschmeidigkeit aufrecht erhalten bleiben. Die Verwendung von Metall, insbesondere Silber, bewirkt die direkte oder verzögerte Abtötung von Bakterien, Pilzen, Viren und Algen durch oligodynamisch wirksames Silber. Vorteilhafterweise findet weiters eine signifikante Reduktion der Interaktionsmöglichkeiten zwischen Keim und Oberfläche durch entsprechende freie Oberflächenenergie statt.

Das Metall ist in einem Filament, einer Faser und/oder einer Spinnfaser oder -faden integriert, wodurch keine separate Beschichtung der Innenauskleidung mit dem metallischen Element durchgeführt werden muss und die antimikrobiellen Eigenschaften somit bereits bei der Herstellung der Innenauskleidung dieser verliehen werden können. Dadurch erspart man sich die Durchführung eines zusätzlichen Arbeitsschritts im Herstellungsprozess der Innenauskleidung.

Das partikelförmige Material weist eine Partikelgröße aus einem Bereich mit einer unteren Grenze vom 1µm, vorzugsweise 0,2µm, insbesondere 20µm und einer oberen Grenze von 500µm, vorzugsweise 250µm, insbesondere 200µm auf, wodurch eine gleichmäßige Beschichtung der Innenauskleidung erzielt werden kann.

Das Metall kann geflockt, geklebt, laminiert, kaschiert, gedampft, gespritzt, getaucht und/oder geflutet werden, wodurch standardisierte Herstellungsprozesse für die Herstellung einer mit Metall beschichteten Innenauskleidung verwendet werden können.

Die zumindest eine weitere Schicht aus semipermeabler Membran kann benachbart, gegebenenfalls unmittelbar benachbart, der zumindest einen Schicht aus einem Gewebe und/oder Gewirke bzw. Leder mit antimikrobieller Wirkung angeordnet sein, wodurch die Effekte der beiden Schichten, nämlich einerseits die wasserabweisenden Eigenschaften der semipermeablen Membran und die antibakteriellen, geruchshemmenden, temperaturregulierenden und antistatischen Eigenschaften der das Metall beinhaltenden Schicht ihre optimale Wirkung entfalten können.

Alternativ kann die zumindest eine Schicht aus einem Gewebe und/oder Gewirke bzw. Leder mit antimikrobieller Wirkung auf die zumindest eine weitere Schicht aus semipermeabler Membran flächig oder partiell, insbesondere punktförmig oder netzartig aufgebracht, wie geflockt, geklebt, laminiert, kaschiert, gedampft, gespritzt, getaucht und/oder geflutet, sein, wodurch die semipermeable Membran direkt auf deren Oberfläche oder sogar integriert in die Membran die antimikrobiellen Eigenschaften aufweist.

In einer alternativen Ausführungsform ist vorgesehen, dass die weitere Schicht aus semipermeabler Membran zwischen der zumindest einen Schicht aus einem Gewebe und/oder Gewirke bzw. Leder mit antimikrobieller Wirkung und einer zusätzlichen Schicht aus einem Gewebe und/oder Gewirke bzw. Leder angeordnet ist, wodurch zusätzlich zu den vorab genannten Eigenschaften noch Eigenschaften, welche die zusätzliche Schicht aufweist, dazu kombiniert werden können. Als Beispiel sei hier eine Funktionalisierung der Innenauskleidung erwähnt, welche zusätzlich Wärmeregulierung, Flammhemmung, Schnittverletzungshemmung oder dergleichen ermöglicht.

In einer Weiterbildung ist vorgesehen, dass die zumindest eine weitere Schicht aus semipermeabler Membran, die zumindest eine Schicht aus einem Gewebe und/oder Gewirke bzw. Leder mit antimikrobieller Wirkung und gegebenenfalls die zusätzliche Schicht aus einem Gewebe und/oder Gewirke bzw. Leder miteinander flächig verbunden sind, wodurch ein Verrutschen der beiden Schichten gegeneinander verhindert werden kann. Die flächige Verbindung der unterschiedlichen Schichten miteinander bedingt außerdem eine gewisse Versteifung und ist damit für bestimmte Anwendungen als Innenauskleidung von Vorteil, weil eine Erhöhung der Formstabilität bewirkt wird.

Weiters erweist sich auch von Vorteil, dass die zumindest eine weitere Schicht aus semipermeabler Membran, die zumindest eine Schicht aus einem Gewebe und/oder Gewirke bzw. Leder mit antimikrobieller Wirkung und gegebenenfalls die zusätzliche Schicht aus einem Gewebe und/oder Gewirke bzw. Leder zumindest über einen Teil der Fläche rutschfest miteinander verbunden sind, wodurch einerseits der Tragekomfort nicht beeinflusst wird und andererseits auch ein gewisses Maß an Formstabilität der Innenauskleidung beibehalten werden kann.

In einer Weiterbildung der erfindungsgemäßen Innenauskleidung ist vorgesehen, dass wenigstens die zumindest eine Schicht aus einem Gewebe und/oder Gewirke bzw. Leder mit antimikrobieller Wirkung und die zumindest eine weitere Schicht aus semipermeabler Membran und gegebenenfalls die zusätzliche Schicht aus einem Gewebe und/oder Gewirke bzw. Leder zumindest partiell, insbesondere an ihren Kontaktflächen in Form einer Vielzahl von Punkten oder netzartig, gegebenenfalls aktivierbaren Haftschicht, miteinander verbunden sind, wodurch sich vorteilhafter Weise die Arbeitsabläufe nicht behindern und die zu verarbeitenden, d.h. aus zugeschnittenen und zusammengefügten Schnittteilen bestehenden Schichten, flächendeckend aufeinander angeordnet und ausgerichtet werden können. Das Haftmittel kann anschließend, beispielsweise durch Hitze oder durch Druck, aktiviert werden und somit die einzelnen Schichten, ob bereits bearbeitet oder nicht, in gewünschter Weise miteinander verbunden werden.

Die Schichten können beispielsweise durch schweißen, heiß siegeln, laminieren und/oder nähen entlang von Verbindungslinien miteinander verbunden werden, wodurch die wasserundurchlässige, allerdings schweißdurchlässige Eigenschaft der semipermeabler Membran und die geruchshemmende, antibakterielle, temperaturregulierende und antistatische Wirkung der Schicht mit Metall aufrecht erhalten werden kann.

Die Innenauskleidung besteht aus wenigstens je einer Schicht von miteinander an Verbindungslinien verbundenen Schnittteilen wenigstens eines Gewebes und/oder Gewirkes bzw. Leder mit antimikrobieller Wirkung einerseits und wenigstens einer weiteren Schicht aus semipermeabler Membran andererseits, wobei die einzelnen Schichten rutschfest und semipermeabel über ihre gesamte gemeinsame Fläche miteinander verbunden sind, wobei die Verbindungslinien der weiteren Schicht aus semipermeabler Membran geschweißt sind, wobei die Verbindungslinien der Teile der Schicht aus einem Gewebe und/oder Gewirke bzw. Leder mit antimikrobieller Wirkung unabhängig von der weiteren Schicht aus semipermeabler Membran miteinander verbunden, vorzugsweise genäht, sind, wodurch jede Schicht mit jenen für sie am geeignetsten Verbindungsmethoden verbunden werden kann.

Vorteilhafterweise ist wenigstens eine Schicht aus einem Gewebe und/oder Gewirke bzw. Leder mit antimikrobieller Wirkung und eine zusätzliche Schicht aus einem Gewebe und/oder Gewirke bzw. Leder angeordnet, wobei die weitere Schicht aus semipermeabler Membran zwischen der Schicht aus einem Gewebe und/oder Gewirke bzw. Leder mit antimikrobieller Wirkung und der zusätzlichen Schicht aus einem Gewebe und/oder Gewirke bzw. Leder angeordnet ist, wobei alle Schichten rutschfest und semipermeabel über ihre gesamte gemeinsame Fläche miteinander verbunden sind und die weitere Schicht aus semipermeabler Membran gemeinsame Verbindungslinien mit zumindest einer der beiden anderen Schichten aufweist, die geschweißt bzw. heißgesiegelt, und vorzugsweise auch genäht sind, wodurch beim Ausziehen des Ausrüstungsgegenstands ein Trennen der einzelnen Schichten voneinander verunmöglicht wird.

Die Innenauskleidung kann ein- oder beidseitig der semipermeabel miteinander verbundenen Schicht(en) aus einem Gewebe und/oder Gewirke bzw. Leder mit antimikrobieller Wirkung und der weiteren Schicht aus semipermeabler Membran wenigstens eine zusätzliche Schicht aus einem Gewebe und/oder Gewirke bzw. Leder und/oder wenigstens eine weitere Schicht aus semipermeabler Membran angeordnet sein, die im wesentlichen über die ganze Fläche rutschfest und semipermeabel mit den semipermeabel verbundenen Schichten aus semipermeabler Membran und aus einem Gewebe und/oder Gewirke bzw. Leder mit antimikrobieller Wirkung verbunden ist, wobei die Verbindungslinien der Teile der weiteren semipermeablen Membran geschweißt sind, während die Verbindungslinien der beiden anderen Schichten aus einem Gewebe und/oder Gewirke bzw. Leder unabhängig von der ersten und/oder der weiteren Membran miteinander verbunden, vorzugsweise genäht, sind, wodurch wiederum eine Perforierung der Membran vermieden werden kann. Wird die Membranschicht beidseitig mit einer Schicht aus Gewebe und/oder Gewirke bzw. Leder in erfindungsgemäßer Weise verbunden, so zeichnet sich ein mit einer derartigen Innenauskleidung ausgestatteter Ausrüstungsgegenstand, insbesondere Kleidungsstück durch besonderen Tragekomfort aus.

Weiters ist vorgesehen, dass die Schichten sowohl ohne als auch mit antimikrobieller Wirkung zumindest teilweise aus Gewebe und/oder Gewirke aus Natur- oder Chemiefasern gebildet sind, wodurch eine sehr vielseitige Ausgestältung und somit viele Verwendungsmöglichkeiten der Innenauskleidung ermöglicht werden. Vorteilhafterweise wird eine große Bandbreite von Fasern mit antimikrobiellen Eigenschaften zur Verfügung gestellt, die auf den Endverbraucher abzielen, wobei die Palette von Sportsocken, Unterwäsche und Handschuhen bis hin zu Schutzbekleidung für Personal in der Gesundheitspflege und bei Noteinsätzen reicht. Der Bedarf an antimikrobiell wirkenden Fasern steigt mit dem Bewusstwerden des allgemeinen Vorteils der besonderen deodorierenden oder zumindest neutralisierenden Eigenschaften derselben Produkte.

Von Vorteil erweist sich dabei, dass die Naturfasern aus einer Gruppe umfassend Pflanzenfasern, insbesondere Pflanzenhaare, wie z.B. Baumwolle, Bastfasern, wie z.B. Flachs, Hanf, Jute, Hartfasern, wie z.B. Kokos, Sisal, Tierfasern, insbesondere Wolle und Haare, wie z.B. Schafwolle, Lama, Rosshaar, Seide, wie z.B. echte Seide, Wildseide, Mineralfasern und/oder Leder ausgewählt sind, wodurch eine sehr gute Saugfähigkeit erzielt werden kann. Vorteilhaft dabei erweist sich auch, dass durch diese Materialien eine sehr gute Isolierung sowohl gegen Hitze als auch gegen Kälte erzielt werden kann.

In einer alternativen Ausführungsform ist vorgesehen, dass Chemiefasern aus natürlichen und/oder synthetischen Polymeren und/oder aus organischen Rohstoffen gebildet sind, wodurch die den Materialien inne wohnenden Eigenschaften der Innenauskleidung verliehen werden können.

Die natürlichen Polymere können pflanzlicher und/oder tierischer Herkunft, wie z.B. Cellulose-, Alginat-, regenerierte Proteinfasern und/oder Elastodien (Gummi) sein, wodurch durch das Herstellungsverfahren dieser Materialien keine bzw. zumindest keine unnatürliche Umweltbelastung verursacht wird.

In einer alternativen Ausführungsform ist vorgesehen, dass die synthetischen Polymere Polymerisat-, wie z.B. Polyacryl, Polychlorid, Polyethylen, Polypropylen, Elastodien, Polykondensat-, wie z.B. Polyamid, Polyester, und/oder Polyadditionsfasern, wie z.B. Polyurethan, Elastan sind, wodurch der Innenauskleidung auch diesen Materialien inne wohnende Eigenschaften, wie z.B. ein gewisses Maß an Dehnbarkeit verliehen werden kann.

Organische Rohstoffe können durch Glasfasern, Metallfasern und/oder Kohlenstofffasern gebildet werden, wodurch der Innenauskleidung eine gewisse Formstabilität bzw. Steifigkeit verliehen werden kann.

Von Vorteil dabei erweist sich, dass die Schichten (3, 6) sowohl ohne als auch mit antimikrobieller Wirkung zumindest teilweise aus Gewebe und/oder Gewirke aus einer Gruppe umfassend Aramid- und/oder Paraaramidfasern, wie Kevlar® (Poly(p-phenylen-terephthalamid), Nomex® (Aramid aus m-Phenylendiamin und Isophthalsäure), Twaron®, Technora, Teijinconex, Phenol-Formaldehydfasern, wie Kynol, Polyamid/Polyimidfasern wie Kermel, Polybenzimidazolfasem oder Fasergemischen daraus, gebildet sind, wodurch beispielsweise flammhemmende und Schnittfeste Eigenschaften erzielt werden können.

Weiters ist vorgesehen, dass mehrere Schichten aus einem Gewebe und/oder Gewirke bzw. Leder mit antimikrobieller Wirkung angeordnet sein können, wodurch somit sowohl an der Innenseite der Innenauskleidung, d.h. jener Seite, welche dem Träger zugewandt ist, eine antimikrobieller Eigenschaft verliehen werden kann, wodurch einerseits eine Geruchsminderung durch das Verhindern von Bakterienwachstum erzielt werden kann und andererseits durch das Anbringen der antimikrobiellen Schicht, beispielsweise auf der Außenseite der Innenauskleidung, auch durch in Kontakt kommen der Innenauskleidung mit pathogenen Stoffen, diese Stoffe ihre Wirkung gar nicht erst entfalten können.

In einer Weiterbildung ist vorgesehen, dass mehrere Schichten aus einem Gewebe und/oder Gewirke bzw. Leder angeordnet sind, wodurch eine höhere Beständigkeit und somit eine längere Lebensdauer der Innenauskleidung erzielt werden kann.

Bei einem Verfahren, mit welchen mehrere Schichten, insbesondere zumindest eine Schicht aus einem Gewebe und/oder Gewirke bzw. Leder mit antimikrobieller Wirkung und eine weitere Schicht aus semipermeabler Membran der gewünschten Innenauskleidung entsprechend in einzelne Schnittteile zugeschnitten und geformt werden, worauf die zumindest zwei Schichten zumindest partiell deckend und gegebenenfalls semipermeabel miteinander verbunden werden, erweist sich von Vorteil, dass eine einem Standardprozedere folgende Assemblierung einer Innenauskleidung erfolgen kann.

Ein Anbringen einer zusätzlichen Schicht aus einem Gewebe und/oder Gewirke bzw. Leder der gewünschten Innenauskleidung entsprechend und in einzelne Schnittteile zugeschnitten und geformt, worauf die Schichten zumindest partiell deckend und gegebenenfalls semipermeabel miteinander verbunden werden, bewirkt vorteilhafter Weise, dass auch mehrere Schichten auf die gleiche Weise zugeschnitten und aneinander gefügt werden können und somit der Arbeitsprozess standardisiert werden kann. Die jeweilige Verbindungsart kann dabei jeweils ganz unabhängig von der anderen Schicht erfolgen und somit ganz der Qualität des Materials der jeweiligen Schicht angepasst werden.

In einer alternativen Ausführungsform erweist sich von Vorteil, dass die zumindest eine Schicht aus einem Gewebe und/oder Gewirke bzw. Leder mit antimikrobieller Wirkung und die zumindest eine Schicht aus semipermeabler Membran und gegebenenfalls eine zusätzliche Schicht aus einem Gewebe und/oder Gewirke bzw. Leder vor dem Zuschneiden zumindest partiell miteinander verbunden werden, worauf diese Schichten gemeinsam zugeschnitten und die Schnittteile der Innenauskleidungsform entsprechend zusammengefügt werden, wodurch die Schnittteile die gewünschten Eigenschaften der einzelnen Bestandteile aufweisen, aber in ihrer Gesamtheit wie eine einzige Schicht wirken, mit gegebenenfalls unterschiedlichen Oberflächen. So ist an der einen Seite der Schicht aus semipermeabler Membran die Schicht mit antimikrobiellen Eigenschaften und an ihrer anderen Seite ein Gewebe und/oder Gewirke bzw. Leder, welches beispielsweise wärmedämmende Eigenschaften, wie z.B. ein wärmedämmender Faserpelz aufweist, angeordnet. Wird nun die aus Geweben und/oder Gewirken bzw. Leder und einer semipermeablen Membranschicht bestehende Innenauskleidung ein- oder beidseitig mit einer die das Gewebe bzw. das Gewirke bzw. Leder ergänzenden Schicht zur antimikrobiellen Schicht verbunden, sind Innenauskleidungen möglich, die eine Kombination von verschiedenartigen, den verwendeten Geweben entsprechenden Eigenschaften, vereinen.

Die Schicht aus einem Gewebe und/oder Gewirke bzw. Leder mit antimikrobieller Wirkung wird gegengleich zur zusätzlichen Schicht aus einem Gewebe und/oder Gewirke bzw. Leder und/oder zur Schicht aus semipermeabler Membran zugeschnitten, wodurch die Nähte, um eine optimale Verbindung der Schicht aus einem Gewebe und/oder Gewirke bzw. Leder mit antimikrobieller Wirkung und der zusätzlichen Schicht bzw. der Schicht aus einem Gewebe und/oder Gewirke bzw. Leder mit antimikrobieller Wirkung, der weiteren Schicht aus semipermeabler Membran und der zusätzlichen Schicht zu gewährleisten, von der weiteren Schicht abgewandten Seite liegen, und somit eine lange Lebensdauer des Ausrüstungsgegenstands bei Aufrechterhaltung voller Funktionalität gegeben ist.

Vorteilhaft dabei erweist sich, dass die Innenauskleidung für Sport-, Freizeit- und/oder Berufskleidung verwendet werden kann, weil eben derartig viele Eigenschaften, wie geruchshemmende, antimikrobielle, temperaturregulierende, antistatische, wasserabweisende, luftdurchlässige, etc. Eigenschaften in der erfindungsgemäßen Innenauskleidung vereint sind und somit eine äußerst vielseitige Anwendung dieser erzielt werden kann.

Von Vorteil erweist sich dabei auch, dass der Ausrüstungsgegenstand als Bekleidung für die oberen Extremitäten, insbesondere Handschuhe und Fäustlinge, für die unteren Extremitäten, wie z.B. Socken, Schuhe, Stiefel, Hosen, Unterwäsche, für den Kopf, wie z.B. Haube, Kappe, Mütze, Maske, und für den Rumpf, wie z.B. Jacke, Mantel, verwendet werden kann, wodurch wiederum die Vielseitigkeit der Innenauskleidung bestätigt wird.

Die Erfindung wird im nachfolgenden anhand der in den Zeichnungen dargestellten Ausführungsbeispiele näher erläutert.

Es zeigen:
- Fig. 1: eine Draufsicht auf einen erfindungsgemäßen Ausrüstungsgegenstand;
- Fig. 2: einen Schnitt durch eine Innenauskleidung mit mehreren Schichten;
- Fig. 3: einen Schnitt durch eine erfindungsgemäße Innenauskleidung mit Verbindungslinien;
- Fig. 4: einen Schnitt durch eine erfindungsgemäße Innenauskleidung in alternativer Ausführung.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

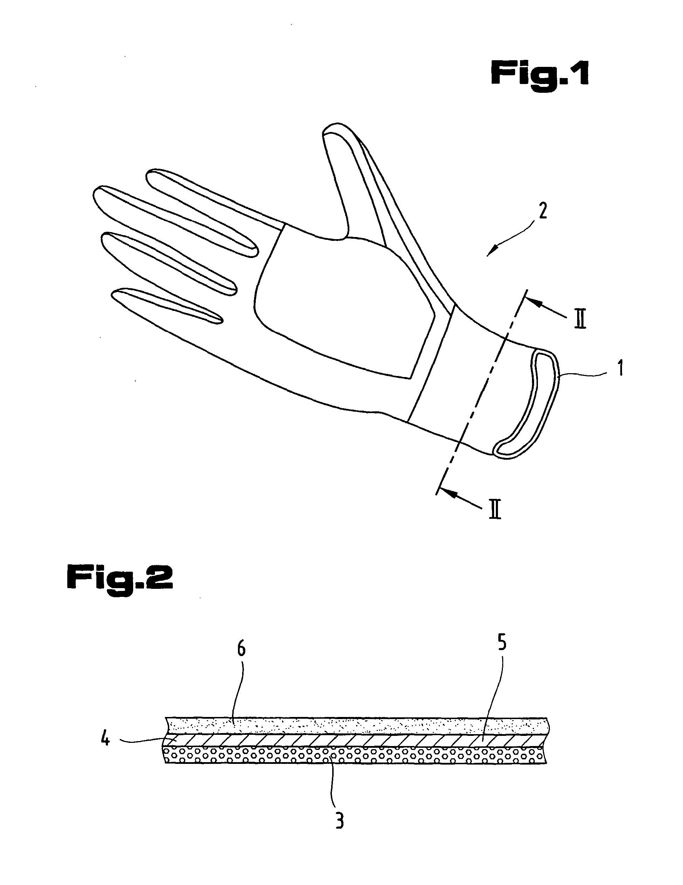

In Fig. 1 ist eine Draufsicht auf einen Ausrüstungsgegenstand dargestellt, wobei auf der Innenseite, d.h. der Hand zugewandten Seite des Ausrüstungsgegenstands 2, die Innenauskleidung 1 angeordnet ist.

An dieser Stelle sei darauf hingewiesen, dass die Innenauskleidung 1 auch die äußerste Schicht eines Ausrüstungsgegenstands 2 bilden kann. Hier sei exemplarisch lediglich erwähnt, dass die Innenseite, beispielsweise eines Golfhandschuhs, aus Leder antimikrobiell ausgestattet sein kann. Alternativ sei auch möglich, dass eine Schicht aus einem Gewebe und/oder Gewirke die äußerste Schicht eines Ausrüstungsgegenstands 2 bildet und antimikrobiell ausgebildet ist.

Die Innenauskleidung 1 für einen Ausrüstungsgegenstand 2 zum zumindest temporären Bedenken von Körperteilen, insbesondere Kleidungsstücke, Decken, Schlafsäcke besteht aus mehreren Schichten.

Fig. 2 zeigt einen Schnitt einer erfindungsgemäßen Innenauskleidung 1, wobei diese aus mehreren Schichten besteht. Eine Schicht 3 aus einem Gewebe und/oder Gewirke bzw. Leder mit antibakteriellen Eigenschaften, eine weitere Schicht 4 beispielsweise aus einer semipermeablen Membran 5, einem Laminat, vorzugsweise aus Polyvinylchlorid oder Polyurethan, und eine zusätzliche Schicht 6 aus einem Gewebe und/oder Gewirke bzw. Leder. Die semi-permeable Membran 5 kann beispielsweise aus Polytetrafluourethylen (PTFE) gebildet sein. Die weitere Schicht 4 kann auch aus Laminat gebildet aus Polyvinylchlorid, Polyurethan, Polyetheramid, Polyetheresteramid, Polyolefinkomponenten, Polyesterkomponenten oder einem Gemisch daraus, gebildet sein. Die Aufzählung der Materialien, welche sowohl Wasser abweisende aber dennoch luftdurchlässige Eigenschaften aufweist soll für die erfindungsgemäße Innenauskleidung 1 nicht limitierend sein.

Eine weitere Schicht 4 aus einer semipermeablen Membran 5 ist benachbart zur Schicht 3 aus einem Gewebe und/oder Gewirke bzw. Leder mit antimikrobieller Wirkung angeordnet. In einer bevorzugten Ausführungsform kann die semipermeable Membran 5 auch unmittelbar benachbart zur Schicht 3 aus einem Gewebe und/oder Gewirke bzw. Leder mit antimikrobieller Wirkung angeordnet sein.

Alternativ kann die semipermeable Membran 5 auch zwischen der zumindest einen Schicht 3 aus einem Gewebe und/oder Gewirke bzw. Leder mit antimikrobieller Wirkung und einer zusätzlichen Schicht 6 aus einem Gewebe und/oder Gewirke bzw. Leder angeordnet sein.

Da die semipermeable Membran 5 beidseitig flächendeckend von Schichten aus einem Gewebe und/oder Gewirke abgedeckt sein kann und die Verbindung mit den einzelnen Schichten rutschfest ist, wirkt das Schichtmaterial in seiner Gesamtheit wie eine Schicht mit den Eigenschaften der dazwischenliegenden Membran 5. In alternativen Ausführungsformen ist einer punktuelle oder netzartige Verbindung der Membran bzw. des Laminats mit der Schichten möglich, wobei ebenfalls ein rutschhemmender Effekt erzielt wird.

Die antimikrobielle Wirkung des Gewebes und/oder Gewirkes bzw. Leders wird durch ein Metall erreicht. Durch die Verwendung eines Metalls, vorzugsweise Silber, wird nicht nur eine antimikrobielle, sondern auch eine antimikrobiotische und antimykotische Wirkung erreicht. Es werden geruchsbildende Bakterien bekämpft und vernichtet und somit wird auch die Entstehung unangenehmen Geruchs verhindert. Die Verwendung eines Metalls ist natürlich, nicht giftig, und enthält keine chemischen Produkte oder Pestizide und ist somit auch ausgesprochen umweltfreundlich.

Des weiteren erweist sich das Metall einerseits im Sommer kühlend und andererseits im Winter wärmend. Der Grund dafür ist, dass Metalle eine extrem große und einmalige Leitfähigkeit besitzen.

Das Metall zur Verwendung für die Innenauskleidung 1 ist aus einer Gruppe umfassend Silber, Gold, Kupfer, Platinium und/oder einem Gemisch daraus, auswählt. In einer bevorzugten Ausführungsform wird Silber verwendet.

Silber weist sehr gute antistatische Eigenschaften auf. Elektrische Schläge, welche durch Statik oder Reibung entstehen können, werden durch die optimale Verteilung der elektrischen Energie in Null-Komma-Nichts verwandelt.

Zudem wird reines Silber verwendet, das als natürliches Element vorkommt. Es bestehen daher keine Vergiftungsrisiken für den Verwender der Innenauskleidung 1. Und auch das allergene Potential reinen Silbers ist gegenüber "verunreinigten" Metallen äußerst gering.

In einer alternativen bevorzugten Ausführungsform wird Gold verwendet, wobei hier die gleichen Eigenschaften wie von Silber zum Tragen kommen und zudem noch ein therapeutischer Effekt, wie er beispielsweise bei der Behandlung von Rheumatikern genutzt werden kann zum Tragen kommt.

Durch die Verwendung von Silber werden nicht nur antimikrobielle bzw. antimikrobiotische sondern auch antigeruchsbildende Eigenschaften der Innenauskleidung 1 erzielt. Die Bakterien sind nämlich nur einer der Gründe zur Bildung von ausströmenden Gerüchen des menschlichen Körpers. Auch Ammoniak und denaturierte Proteine tragen bedeutend zur Bildung von Gerüchen von Innenauskleidungen 1 bei. Sowohl Ammoniak als auch denaturierte Proteine binden sich leicht an Silber. Da die Innenauskleidung 1 auf der Innenseite Silber enthält, ermöglicht es dem Ammoniak und den denaturieren Proteinen rasch chemische Bindungen mit Silber einzugehen und somit die unverzügliche Verringerung von Gerüchen einzuleiten.

Durch die Verwendung von Silber gelingt es auch, die Energieabstrahlung des menschlichen Körpers in der kalten Jahreszeit niedrig zu halten. Die dienlichste Methode diese Energie nutzen zu können, ist es, sie zur Emissionsquelle zurückzuleiten oder sie zu erhalten. Silber bietet zudem eine ideale Lösung gegen Kälte. Die Abhilfen gegen Kälte müssen Probleme wie Ausstrahlung, Verdunstung und Konvektion lösen. Der Reflexfaktor der Infrarotstrahlen von Silber liegt über 95%. Es handelt sich hierbei um Werte, die im Vergleich zu allen Elementen viel höher liegen, wobei dies praktisch gesehen bedeutet, dass 95% der abgegebenen Energie, die mit Silber in Kontakt kommt, an ihren Ursprung zurück reflektiert wird. Silber weist im Vergleich zu anderen Elementen eine der niedrigsten Ausstrahlungsraten auf. Das ist gleich zu setzen mit einer sehr niedrigen Ausstrahlungsgeschwindigkeit von Wärmeenergie. Silber bleibt somit sehr lange warm, im Gegensatz zu anderen Elementen, die für nur sehr kurze Zeit Wärme erhalten können. Innenauskleidungen mit Silber bewirken, dass die Wärme, die nicht auf den Körper reflektiert wird, einen sehr langen Zeitraum von der Innenauskleidung 1 aufgenommen und gespeichert wird, wodurch die Innenauskleidung 1 den Träger für einen längeren Zeitraum warm hält, als im Vergleich mit herkömmlichen Produkte, die mit inaktiven Fasern hergestellt werden.

In der warmen Jahreszeit ist der wesentlichste Mechanismus der Wärmeübertragung eine Energieumwandlung zu ermöglichen und dadurch die Körpertemperatur erniedrigen zu können, wobei Wärmeableitung an die Umgebung erstrebenswert ist. Wenn es nicht möglich ist, Wärme durch Leitung oder Wärmeströmung ausreichend abzugeben, bedient sich der Körper der Verdunstung. In diesem Fall ist das Ergebnis Schweiß. Um das Wohlbefinden des Körpers gewährleisten zu können ist es unerlässlich, die sich so bildende Feuchtigkeit fern zu halten, wie dies durch die Verwendung einer semipermeablen Membran 5 in Kombination mit dem Gewebe und/oder Gewirke bzw. Leder mit antimikrobiellen Eigenschaften bewirkt wird.

Die Wärmeübertragung durch Konvektion kann zudem erhöht oder verringert werden, je nachdem, ob eine oder keine isolierenden bzw. windabweisenden Materialien eingesetzt werden, wie dies ebenfalls durch die Verwendung der semipermeablen Membran 5 erzielt werden kann. Durch die Verwendung der semipermeablen Membran 5 wird zudem eine vielseitige Anwendung der Innenauskleidung 1 ermöglicht. Die semipermeable Membran ermöglicht es, den Körper des Trägers der Innenauskleidung 1 vor eintretender Nässe zu schützen bzw. den Träger vor widrigen Wind- und Wetterverhältnissen zu schützen.

Durch die Verknüpfung der Eigenschaften der semipermeablen Membran 5 und des Silbers ermöglicht die Innenauskleidung 1 sowohl ein trocken halten des Trägers als auch eine Temperaturregulation. Der Träger schwitzt nicht und stinkt somit auch nicht.

Wird Silber mit anderen wasserabweisenden Materialien in eine feuchte Umgebung gebracht, erhöhen die Leitungseigenschaften die Verdunstungsgeschwindigkeit der Feuchtigkeit. Während diese Feuchtigkeit durch Verdunstung umgewandelt wird, kann eine noch größere Menge an Feuchtigkeit der wasserabweisenden semipermeablen Membran 5 entgegengebracht und somit verdunstet werden. Demzufolge beseitigen Innenauskleidungen 1 mit Silber Körperfeuchtigkeit schneller, schaffen somit eine behaglichere Umgebung und verringern außerdem den Verlust durch Konvektion.

Während sich die Temperatur erhöht, verliert die Ausstrahlung an Wichtigkeit. Einer der Hauptgründe dafür ist die Wärmeübertragung. Silber nimmt es mit der Wärmeleitung und der Verdunstung auf. Silber hat im Vergleich zu allen anderen Elementen die höchste Fähigkeit der Wärmeleitung. D.h. Innenauskleidungen 1 mit Silber wirken aktiv, um die Hitze schnell und gleichmäßig auf die gesamte Innenauskleidung 1 zu verteilen. Während der warmen Jahreszeit, wenn Wärmeleitung als hauptsächliches Mittel der Wärmeumwandlung dient, fördert eine Innenauskleidung 1 mit Silber die natürliche Körperfunktion, in dem die Wärmeleitung, die von der Haut ausgeht, erhöht wird. Wobei im Gegensatz dazu ein herkömmlicher Stoff lediglich der Wärmeleitung als Barriere entgegen steht. Silber leitet die Hitze der Haut an die Umgebung ab und vergünstigt somit eine Abkühlung des Körpers. Durch die Weiterleitung der Feuchtigkeit durch Silber wird auch die Feuchtigkeit auf der Haut auf ein Minimum herabgesetzt und das Wohlbefinden des Körpers erhöht.

Eine Innenauskleidung 1 mit Silber kann zudem antistatisch wirken und sowohl in der Industrie als auch bei der Anwendung in Konsumgütern einen großen Vorteil erzielen. Für die meisten Menschen ist ein elektrischer Schlag durch Statik oder die Reibung des Stoffes auf der Haut unangenehm. In gewissen industriellen Anwendungen kann dieses Phänomen zudem eine große Gefahr mit sich bringen.

Statische Elektrizität entsteht durch die statische Entladung oder durch Reibungsenergie. Bei Innenauskleidungen 1 aus synthetischen Materialien kann eine sehr hohe Ladung erzeugt werden. Die einzige Methode, um diese Unannehmlichkeiten zu verringern oder zu beseitigen, besteht in der Verteilung der elektrischen Ladung durch leitende Materialien. Im Vergleich zu allen anderen Elementen ist Silber das mit der höchsten Leitfähigkeit. Schon sehr geringe Mengen an Silber in der Innenauskleidung 1 ermöglichen es, elektrische Reibungsaufladungen zu verteilen und die somit unangenehmen und elektrischen Aufladungen zu verhindern.

Silber, Gold, Platinium, Kupfer, etc. in Innenauskleidungen 1 weisen zudem noch therapeutische Eigenschaften auf. Dies basiert meist auf dem Einsatz der elektrischen und thermischen Leitfähigkeit von Silber. Der menschliche Körper ist mit Tausenden von Nervensträngen ausgestattet, die an der Oberfläche der Haut enden. Der Körper benutzt diese Nerven, um verschiedene elektrische Signale auszulösen, wie z.B. Wahrnehmungen von Schmerz und Unbehagen. Da Silber das Material mit der höchsten Leitfähigkeit ist, sammeln Innenauskleidungen 1 mit Silber diese elektrischen Ladungen, die sich auf der Haut befinden und verteilen sie und verringern somit das Gefühl von Unbehagen.

Die elektrischen Strömungen, die im menschlichen Körper vorhanden sind und durch eine Innenauskleidung 1 mit Silber verteilt werden, bilden ein magnetisches Feld rings um den Körper, wobei jedes mal, wenn Elektrizität durch den Körper geht, ein magnetisches Feld entsteht. Neben der weit verbreiteten Überzeugung, die eine Magnettherapie als gesundheitliche Wohltat empfiehlt, bestehen auch veröffentliche Forschungen angesehener gesundheitlicher Einrichtungen, die mit den Auslegungen hinsichtlich der Fähigkeit von Produkten mit magnetischen Eigenschaften übereinstimmen, welche die Blutzirkulation antreiben und beispielsweise eine Verringerung von Anschwellungen bewirken. Somit ergibt sich durch die erfindungsgemäße Innenauskleidung 1 auch ein medizinisch nachweisbar positiver Effekt auf den Träger.

Das Metall, insbesondere Silber, beträgt einen Prozentanteil mit einer oberen Grenze von 80%, vorzugsweise 35%, insbesondere 20% und einer unteren Grenze von 0,5%, vorzugsweise 1%, insbesondere 5% in der Schicht (3), aus Gewebe und/oder Gewirke bzw. Leder der Innenauskleidung 1. In einer bevorzugten Ausführungsform werden 6 bis 20 % Silber in der Schicht 3 verwendet. Trotz der Verwendung des Silbers für die Innenauskleidung 1 werden der Tragekomfort und die ursprünglichen Textileigenschaften und die Tastgeschmeidigkeit aufrecht erhalten.

Es wird reine Metalle verwendet, weil dadurch die Auslösung von Allergien am unwahrscheinlichsten ist.

Das Metall wird in der Innenauskleidung 1 in einem Filament, in einer Faser, einem Spinnfaden bzw. -fasern angeordnet. Die Filamente, Fasern, Spinnfaden bzw. Spinnfasern mit Metall, die für die Herstellung des Gewebes und/oder Gewirkes verwendet werden, werden in jener Form in das Gewebe verarbeitet, das an der Innenfläche bzw. an der Kontaktfläche der Innenauskleidung 1 mit der Haut des Trägers das Silber zu liegen bekommt und dadurch die Eigenschaften des Silbers optimal ausnützen zu können. Die Verbindung des Silbers mit dem Filament, Faser, und/oder Spinnfaser bzw. Spinnfaden ist dauerhaft und irreversibel. Es wäscht sich nicht aus, und trägt sich auch nicht ab.

Alternativ kann das Metall in Form einer Beschichtung oder eines Puders auf die zumindest eine Schicht (3) aus einem Gewebe und/oder Gewirke bzw. Leder und/oder weitere Schicht (4), vorzugsweise aus semipermeabler Membran (5), aufgebracht werden. Dieses Beispiel soll lediglich das Verständnis der Erfindung erleichtern und ist nicht Teil der Erfindung.

Bei der partikelförmigen Aufbringung des Metalls kann die Partikelgröße aus einem Bereich mit einer unteren Grenze von 0,2µm, vorzugsweise 5µm, insbesondere 20µm und einer oberen Grenze von 500µm, vorzugsweise 250µm, insbesondere 200µm ausgewählt sein. In alternativen Ausführungsformen ist es auch denkbar, dass das Metall geflockt, geklebt, laminiert, kaschiert, gedampft, gespritzt, getaucht und/oder geflutet oder dgl. Wird.

In einer Weiterbildung der Innenauskleidung ist die zumindest eine Schicht 3 aus einem Gewebe und/oder Gewirke bzw. Leder mit antimikrobieller Wirkung auf die zumindest eine weitere Schicht 4 aus semipermeabler Membran 5 flächig oder partiell, insbesondere punktförmig oder netzartig aufgebracht, wie geflockt, geklebt, laminiert, kaschiert, gedampft, gespritzt, getaucht und/oder geflutet. Die weitere Schicht 3 muss also nicht notwendigerweise als vollflächig ausgebildet sein, sondern kann in bestimmten mehr oder weniger regelmäßigen Strukturen angeordnet sein. Wie bereits erwähnt kann die Schicht 3 auch auf die weitere Schicht 4 beispielsweise aufgeflockt werden bzw. bei deren Herstellung bereits direkt integriert werden und somit sind die antimikrobiellen und semipermeablen Eigenschaften in einer Schicht miteinander vereint.

Für Innenauskleidungen von Kleidungsstücken, wie beispielsweise von Handschuhen, bei denen eine Vielzahl von Schnittteilen untereinander zu verbinden ist, können die Schnittteile der weiteren Schicht 4 aus semipermeabler Membran 5 in bekannter Weise, beispielsweise durch Schweißen, miteinander verbunden werden, wohingegen die Schnittteile der Schicht 3, 6 aus einem Gewebe und/oder Gewirke bzw. Leder mit oder ohne antimikrobieller Wirkung in üblicher, arbeitssparender Weise, miteinander vernäht werden können. Diese Schichten 3, 4, 6, die über ihre gesamte gemeinsame Fläche semipermeabel miteinander verbunden sind, haften rutschfest aufeinander, die Innenauskleidung als ganze weist somit die erwünschten Eigenschaften der Membranschicht 5 als auch der Schicht 3 mit antimikrobieller Wirkung selbst auf.

Alternativ - wenn auch arbeitsaufwendiger - können Membran- und andere Schichten aus Gewebe und/oder Gewirke bzw. Leder zuerst über ihre gesamte Fläche rutschfest und semipermeabel verbunden werden, dann in die erforderlichen Teile geschnitten und in die gewünschte Form zusammengefügt, beispielsweise genäht, werden, wonach diese Verbindungslinien, um allfällige Perforationen in der Membran abzudichten, nachgeschweißt bzw. versiegelt werden. Eine weitere, aus gegengleich verbundenen, vorzugsweise zusammengenähten, Schnittteilen bestehende, Schicht aus Gewebe und/oder Gewirke bzw. Leder wird daraufhin mit der anderen Membranseite, wiederum über die gesamte gemeinsame Fläche rutschfest und semipermeabel verbunden. Diese etwas aufwendigere Verarbeitungsart kann aber für bestimmte, beispielsweise besonders feste Nähte erfordernde, Kleidungsstücke von Vorteil sein. Auch kann auf bereits vorgefertigtes Material, das aus einer Schicht 4 aus semipermeabler Membran 5 und wenigstens einer an einer Seite mit dieser verbundenen Schicht 3, 6 aus einem Gewebe und/oder Gewirke mit oder ohne antimikrobieller Wirkung zurückgegriffen werden.

Die eine Schicht 3 aus einem Gewebe und/oder Gewirke bzw. Leder mit antimikrobieller Wirkung und die weitere Schicht 4 aus der semipermeablen Membran 5 sind miteinander flächig verbunden. In einer bevorzugten Ausführungsform sind diese beiden Schichten 3, 4 unmittelbar miteinander flächig verbunden, wobei diese Verbindung nicht vollflächig sondern auch nur partiell bzw. punkt- oder gitterförmig erfolgen kann. Dadurch wird bewirkt, dass die beiden Schichten 3, 4 zumindest über einen Teil der Fläche rutschfest miteinander verbunden sind.

Auch die eine Schicht 3 aus einem Gewebe und/oder Gewirke bzw. Leder mit antimikrobieller Wirkung und die zumindest weitere Schicht 4 aus semipermeabler Membran und die zusätzliche Schicht 6 aus einem Gewebe und/oder Gewirke bzw. Leder können unmittelbar miteinander flächig verbunden sein. Auch diese Schichten 3, 4, 6 können in einer bevorzugten Ausführungsform über einen Teil der Fläche rutschfest miteinander verbunden sein.

Die einzelnen Schichten 3, 4, 6 können auch flächendeckend bzw. rutschfest miteinander verbunden werden, wobei die einzelnen Schichten 3, 4, 6 in ihrer Gesamtheit somit wie ein Gewebe und/oder Gewirke wirken, wie das den Eigenschaften der aus dem Gewebe und/oder Gewirke bestehenden Materialien entspricht.

Die zumindest eine Schicht 3 aus einem Gewebe und/oder Gewirke bzw. Leder mit antimikrobieller Wirkung ist mit der weiteren Schicht 4 aus der semipermeablen Membran zumindest partiell, insbesondere an ihrer Kontaktfläche in Form einer Vielzahl von Punkten, gegebenenfalls aktivierbaren Haftschicht miteinander verbunden. In einer alternativen Ausführungsform ist auch die zusätzliche Schicht 6 aus einem Gewebe und/oder Gewirke bzw. Leder mit der Schicht 3 aus einem Gewebe und/oder Gewirke bzw. Leder mit antimikrobieller Wirkung und der weiteren Schicht 4 aus der semipermeablen Membran 5 zumindest partiell oder an ihrer Kontaktfläche in Form einer Vielzahl von Punkten miteinander verbunden. Auch diese Verbindung kann über eine aktivierbare Haftschicht erfolgen.

Die vorliegende Erfindung beschreibt auch die Verbindungsart der Schicht 3 aus einem Gewebe und/oder Gewirke bzw. Leder mit antimikrobieller Wirkung mit der weiteren Schicht 4 aus semipermeabler Membran 5. Die Verbindungsart entspricht jener der EP 0 646 061 B1.

Zur Verbindung der einzelnen Schichten 3, 4, 6 kann das partiell aufzubringende Haftmittel vor deren Verarbeitung auf die einzelnen Schichten aufgebracht werden. Dazu erweist sich insbesondere ein aktivierbares Haftmittel von Vorteil. D.h. aus zugeschnittenen und zusammengefügten Schnittteilen bestehende Schichten können flächendeckend aufeinander angeordnet und ausgerichtet werden. Das Haftmittel wird erst anschließend aktiviert, beispielsweise durch Hitze oder auch Druck, die einzelnen Schichten, ob bereits bearbeitet oder nicht, werden in gewünschter Weise miteinander verbunden. Alternativ können bei der Aktivierung des Haftmittels auch partikelförmige Metalle, welche mit einem Harz versehen sind, gleichzeitig mit der aktivierbaren Haftschicht durch die Anwendung von Hitze oder durch Druck ebenfalls auf das Gewebe und/oder Gewirke bzw. Leder aufgebracht werden und somit die antimikrobielle Eigenschaften der Innenauskleidung 1 verliehen werden.

Das Haftmittel, aktivierbar oder sofortklebend, kann aber auch erst nach der Verarbeitung, dem Zusammenfügen von Schnittteilen beispielsweise aufgetragen, z.B. aufgesprüht, werden.

Die Haftschicht kann entweder auf die Schicht 3 aus einem Gewebe und/oder Gewirke mit antimikrobieller Wirkung oder auf die weitere Schicht 4 aus semipermeabler Membran 5 aufgetragen werden, jedoch wird das Auftragen auf die Schicht 3 vorzuziehen sein, da es im selben Arbeitsgang wie das Versehen mit einem Verbindungsmittel erfolgen kann. Die Verbindungsmittel und die Haftschicht könnten somit in gleicher Weise, streifen- oder gitterförmig, oder in Form einer Vielzahl von Punkten aufgetragen werden.

Das Verbindungsmittel kann eine aufgebrachte Kaschiermasse oder eine Haftschicht sein. Wird es vollflächig aufgetragen, so ist wesentlich, dass es permeabel wirkt wie die semipermeable Membran 5 selbst. Die Verbindung kann wie bereits erwähnt auch nur partiell über Fasern, wie sie beispielsweise bei Flammkaschierung erreicht wird, erfolgen.

Die Verbindung der einzelnen Schichten 3, 4, 6 kann beispielsweise auch durch Kleben, Schweißen, Heißsiegeln, Laminieren, Nähen entlang der Verbindungslinien, etc. erfolgen.

Ist die Schicht 4 aus semipermeabler Membran 5 an ihren beiden Flächen mit einer Schicht 3, 6 aus einem Gewebe und/oder Gewirke mit oder ohne antimikrobieller Wirkung verbunden, so besitzt die Innenauskleidung 1 in ihrer Gesamtheit die gewünschten Eigenschaften der semipermeablen Membran 5, wirkt aber wie eine einzige Schicht 3, 6 aus einem Gewebe und/oder Gewirke bzw. Leder, mit gegebenenfalls unterschiedlichen Oberflächen. So kann an einer Seite der Schicht 4 ein Vlies vorgesehen sein, an ihrer anderen Seite aber ein wärmedämmender Faserpelz.

Wird die aus einer Schicht 3 aus einem Gewebe und/oder Gewirke bzw. Leder mit antimikrobieller Wirkung und einer Schicht 4 aus semipermeabler Membran 5 bestehende Innenauskleidung 1 ein- oder beidseitig mit einer weiteren Schicht 6 aus einem Gewebe und/oder Gewirke bzw. Leder oder auch einer weiteren Schicht 4 aus semipermeabler Membran 5 verbunden, sind Innenauskleidungen 1 möglich, die eine Kombination von verschiedenartigen, den Schichten 3, 6 aus einem Gewebe und/oder Gewirke bzw. Leder, bzw. den unterschiedlichen Membranen zuordenbaren Eigenschaften denkbar machen, wobei jede dieser Schichten, ob Schicht 3, 6 aus einem Gewebe und/oder Gewirke bzw. Leder oder Schicht 4 aus semipermeabler Membran 5 aus miteinander verbundenen Schnittteilen besteht. Die jeweiligen Verbindungsart kann dabei jeweils ganz unabhängig von der einer anderen Schicht erfolgen, und ganz der Qualität des jeweiligen Materials angepasst werden.

Die Haftschicht kann für ein Schichtmaterial, das in Bahnen weiterverarbeitet werden soll, wie beispielsweise für Schlafsack-Innenauskleidungen 1, sofortklebend sein, so dass beim Kaschieren von zwei Schichten 3, 6 aus einem Gewebe und/oder Gewirke im selben Arbeitsgang auch das Verbinden mit der semipermeablen Membran 5 vor sich gehen kann. Werden allerdings, wie beispielsweise bei Handschuh-Innenauskleidungen 1, aufwendigere Verarbeitungsschritte mit vielfältig zu verbindenden Schnittteilen notwendig, so wird eine Haftschicht, die erst aktiviert werden muss, vorzuziehen sein.

Die Schicht 3 aus einem Gewebe und/oder Gewirke bzw. Leder mit antimikrobieller Wirkung und die zusätzliche Schicht 6 aus einem Gewebe und/oder Gewirke bzw. Leder kann zumindest teilweise aus Natur- und/oder Chemiefasern gebildet sein. Die Naturfasern können aus einer Gruppe umfassend Pflanzenfasern, insbesondere Pflanzenhaare, wie z.B. Baumwolle, Bastfasern, wie z.B. Flachs, Hanf, Jute, Hartfasern, wie z.B. Kokossisal, Tierfasern, insbesondere Wolle und Haare, wie z.B. Schafwolle, Lama, Rosshaar, Seide, wie z.B. echte Seide, Wildseide, Mineralfasern und/oder Leder ausgewählt sein.

Die Chemiefasern können aus natürlichen und/oder synthetischen Polymeren und/oder organischen Rohstoffen gebildet sein, wobei die natürlichen Polymere pflanzlicher und/oder tierischer Herkunft, wie z.B. Zellulose, Alginat, regenerierte Proteinfasern und/oder Elastodien (Gummi), umfassen können.

Die synthetischen Polymere können Polymerisat-, wie z.B. Polyacryl, Polychlorid, Polyethylen, Polypropylen, Elastodien, Polykondensat-, wie z.B. Polyamid, Polyester und/oder Polyadditionsfasem, wie z.B. Polyurethan, Elastan, sein.

Als organische Rohstoffe können z.B. Glasfasern, Metallfasern und/oder Kohlenstofffasern für die Herstellung der Schicht 3 aus einem Gewebe und/oder Gewirke bzw. für die zusätzliche Schicht 6 aus einem Gewebe und/oder Gewirke verwendet werden.

Um flammhemmende und schnittfeste Eigenschaften der Innenauskleidung 1 zu erzielen können die Schichten (3, 6) sowohl ohne als auch mit antimikrobieller Wirkung zumindest teilweise aus Gewebe und/oder Gewirke aus einer Gruppe umfassend Aramid- und/oder Paraaramidfasem, wie Kevlar® (Poly(p-phenylen-terephthalamid), Nomex® (Aramid aus m-Phenylendiamin und Isophthalsäure), Twaron®, Technora, Teijinconex, Phenol-Formaldehydfasem, wie Kynol, Polyamid/Polyimidfasern wie Kermel, Polybenzimidazolfasern oder Fasergemischen daraus gebildet sein.

In alternativen Ausführungsformen ist es selbstverständlich denkbar, dass mehrere Schichten eines Typs angeordnet sind, d.h. es können sowohl mehrere Schichten 3 aus einem Gewebe und/oder Gewirke bzw. Leder mit antimikrobieller Wirkung angeordnet sein. Alternativ ist auch möglich, dass mehrere Schichten der zusätzlichen Schicht 6 aus einem Gewebe und/oder Gewirke bzw. Leder angeordnet sind.

Bei der Verbindung mehrerer Schichten ist allerdings darauf zu achten, dass die Eigenschaften der semipermeablen Membran 5 und die Eigenschaft der Schicht 3 des Gewebes und/oder Gewirkes bzw. Leders mit den antimikrobiellen Eigenschaften nicht durch die Art der Kaschierung in negativer Weise beeinflusst wird. Es ist dabei darauf zu achten, dass das Verbindungsmittel, z.B. das Haftmittel, zwischen Membran und Gewebe und/oder Gewirke bzw. Lederschicht zwar gleichmäßig verteilt, aber nur partiell, oder selbst semipermeabel auf die jeweilige Schicht aufgetragen wird. Das Haftmittel, aktivierbar oder sofortklebend, kann aber auch erst nach der Verarbeitung, dem Zusammenfügen von Schnittteilen, aufgetragen, z.B. aufgesprüht werden.

Bei sehr hoher Beanspruchung durch Wind und Wetter ist es sogar denkbar, dass mehrere Schichten 4 aus semipermeabler Membran 5 angeordnet sind.

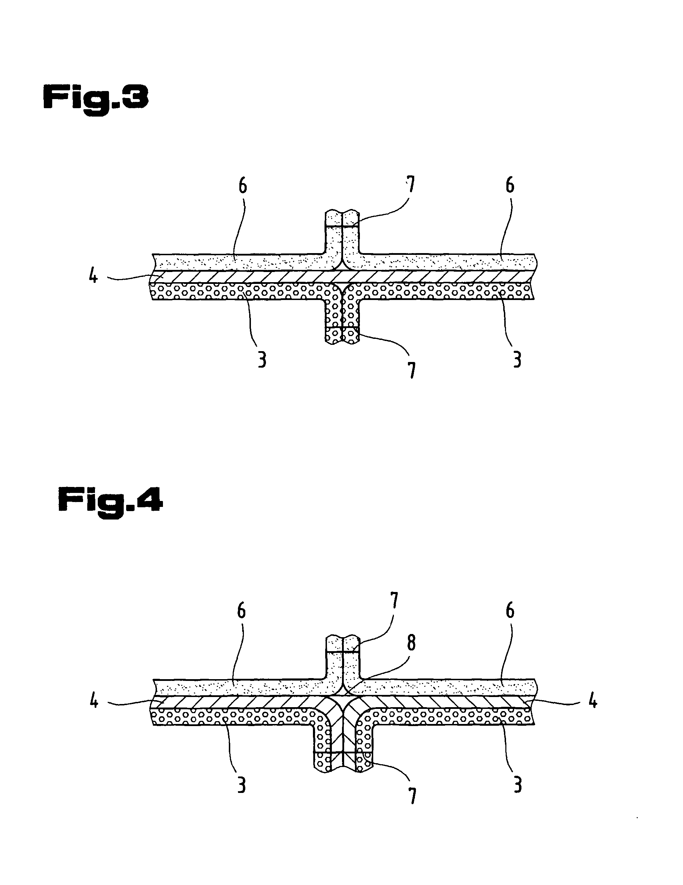

In Fig. 3 ist ein Schnitt durch eine erfindungsgemäße Innenauskleidung 1 dargestellt. Dazu wird die weitere Schicht 4 aus semipermeabler Membran 5 und die Schichten 3, 6 aus Gewebe und/oder Gewirke bzw. Leder in die erforderlichen Schnittteile geschnitten. Die Schnittteile werden daraufhin in bekannter Weise, beispielsweise unter Bildung einer Schweißverbindung, zusammengefügt. Damit bleibt die semipermeable Membran 5 unverletzt, da sie nicht wie im Nähen durchstochen wird, und die Innenauskleidung 1 ist somit als ganzes einwandfrei semipermeabel. Die Schichten 3, 6 aus Gewebe und/oder Gewirke bzw. Leder werden in üblicher Weise an der Verbindungslinie 7 zusammengenäht und bilden somit Schichten der Innenauskleidung 1. Danach werden die resultierenden, einzelnen Schichten 3, 4, 6 zumindest partiell flächendeckend und korrekt ausgerichtet übereinander angeordnet, bei der Herstellung eines Handschuhs vorzugsweise über ein Handmodell gestülpt und entweder von außen und/oder - über ein heizbares Handmodell - von innen erwärmt, sodass die Haftschichten zwischen der Schicht 4 aus semipermeabler Membran 5 und de Schichten 3, 6 aus einem Gewebe und/oder Gewirke bzw. Leder mit oder ohne antimikrobieller Wirkung aktiviert werden. Alle drei Schichten 3, 4, 6 bilden nun eine einzige Innenauskleidung 1 mit semipermeabler Eigenschaft bei textiler Qualität.

Da die Verbindungslinien 7 an den Schichten 3, 6 der Innenauskleidung 1, um auch gerade an den aneinandergrenzenden Flächen der Schichten 3, 6 eine bestmögliche Klebeverbindung mit der Schicht 4 aus semipermeabler Membran 5 zu gewährleisten, in der in Fig. 3 dargestellten Art von der Membran 5 abgewandt liegen sollten, muss beim Zuschneiden der sich jeweils entsprechenden Teile de Innenauskleidung 1 darauf geachtet werden, dass diese gegengleich zugeschnitten werden.

Fig. 4 zeigt eine Alternative zu dieser Innenauskleidung 1, bei der nicht - und damit unterschiedlich zu Fig. 3 - die Schicht 4 aus semipermeabler Membran 5 und die Schichten aus Gewebe und/oder Gewirke bzw. Leder mit oder ohne antimikrobieller Wirkung unabhängig voneinander in die erforderlichen Schnittteile geschnitten werden. Es wird hier von einem der Fig. 1 entsprechenden Schichtmaterial ausgegangen, bei dem die semipermeable Membran 5 und wenigstens eine Schicht 3, 6 aus einem Gewebe und/oder Gewirke bzw. Leder über einen Haftstoff in oben geschriebener Weise semipermeabel über ihre gesamte Fläche miteinander verbunden sind. Dieses Schichtmaterial wird dann in die erforderlichen Schnittteile geschnitten und an einer Verbindungslinie 7 zusammengefügt, beispielsweise genäht. Da dabei Perforationen in der semipermeablen Membran 5 entstehen können, wodurch deren Semipermeabilität vermindert wird, müssen diese Verbindungslinien 7 noch zusätzlich durch eine Abdichtung 8 geschweißt bzw. versiegelt werden. Die Schicht 3 aus einem Gewebe und/oder Gewirke bzw. Leder mit antimikrobieller Wirkung allerdings wird für sich - gegebenenfalls gemeinsam mit einer zusätzlichen Schicht 6 aus einem Gewebe und/oder Gewirke bzw. Leder, die beispielsweise aufkaschiert ist in Schnittteile geschnitten, die an der Verbindungslinie 7 miteinander verbunden, beispielsweise genäht, werden. Die Verbindungslinie 7 weist dabei vorteilhafterweise nach außen, von der semipermeablen Membran 5 weg, sodass bei der Verbindung der beiden Innenauskleidungsschichten 3, 6 mittels des Haftstoffes auch und gerade an der allen Schichtschnittteilen gemeinsamen Verbindungslinie 7 eine einwandfreie Haftung bei gleichzeitiger Wahrung der Semipermeabilität des Innenfutters als ganzes gewährleistet ist. Die fertige Innenauskleidung 1 wird dann, beispielsweise in den Handschuh, gezogen.

Kommt zwar bei den in Fig. 3 und 4 dargestellten Innenauskleidungen 1 jeweils die Schicht 3, 6 aus einem Gewebe und/oder Gewirke bzw. Leder mit der Innenseite des Handschuhs in Kontakt, womit beim Ausziehen des Handschuhs das gleichzeitige Herausziehen der Innenauskleidung 1 erheblich erschwert wird, so wird dies über eine zusätzlich an der Schicht 3, 6 aus einem Gewebe und/oder Gewirke bzw. Leder in beschriebener Weise aufgebrachte Haftschicht, die beispielsweise nach Aktivierung den Außen-Handschuh und die Innenauskleidung 1 fest verbindet, verunmöglicht.

Ein breiter Spielraum besteht in der Auswahl und Zusammensetzung der einzelnen Schichten der Innenauskleidung 1, die wie bereits beschrieben, auch untereinander kombiniert werden können.

Eine weitere Anwendungsmöglichkeit besteht darin, dass eine derartige Innenauskleidung 1 aus mehreren Schichten bestehen kann, so ist auch die Kombination unterschiedlicher Membrane mit verschiedenen Eigenschaften denkbar.

Die Erfindung umfasst zudem ein Verfahren zur Herstellung einer Innenauskleidung, in welchem mehrere Schichten, insbesondere zumindest eine Schicht 3 aus einem Gewebe und/oder Gewirke bzw. Leder mit antimikrobieller Wirkung und eine weitere Schicht 4 aus semipermeabler Membran 5 der gewünschten Innenauskleidung 1 entsprechend in einzelne Schnittteile zugeschnitten und geformt, insbesondere jede Schicht 3, 4 für sich verarbeitet werden, worauf die zumindest zwei Schichten 3, 4 zumindest partiell miteinander verbunden werden können.

In einer Weiterbildung des Verfahrens ist es möglich, dass zu den Schichten 3 und 4 die zusätzliche Schicht 6 aus einem Gewebe und/oder Gewirke bzw. Leder der gewünschten Innenauskleidung 1 entsprechend in einzelne Schnittteile zugeschnitten und geformt wird, worauf die zumindest drei Schichten 3, 4, 6 zumindest partiell deckend miteinander verbunden werden.

Bei alternativen Ausführungsformen der Erfindung ist es auch möglich, dass mehrere als die genannten drei Schichten 3, 4, 6 der Innenauskleidung 1 entsprechend in einzelne Schnittteile zugeschnitten und geformt werden und diese Schichten zumindest partiell deckend miteinander verbunden werden.

Alternativ ist es auch möglich, dass die zumindest eine Schicht 3 aus einem Gewebe und/oder Gewirke bzw. Leder mit antimikrobieller Wirkung und die weitere Schicht 4 aus semipermeabler Membran 5 vor dem Zuschneiden zumindest partiell miteinander verbunden werden, worauf diese Schichten 3, 4 gemeinsam zugeschnitten und die Schnittteile der Innenauskleidungsform entsprechend zusammengefügt werden.

Wie bereits oben erwähnt, kann auch eine zusätzliche Schicht 6 aus einem Gewebe und/oder Gewirke bzw. Leder mit den beiden Schichten 3 und 4 vor dem Zuschneiden zumindest partiell miteinander verbunden werden, worauf diese drei Schichten 3, 4, 6 gemeinsam zugeschnitten werden und die Schnittteile der Innenauskleidungsform entsprechend zusammengefügt werden.
Die Innenauskleidung 1 kann für die Herstellung von Handschuhen, Fäustlingen, wobei der erste Finger und die Finger 2 bis 5 in jeweils einer Einheit, oder Finger 1, Finger 2, und Finger 3, 4, und 5 in jeweils einer Einheit oder Finger 1, Finger 2 und 3, und Finger 4 und 5 in jeweils einer Einheit stecken, Mützen, Hauben, Kappen, Socken, Schuhen, Stiefeln, Jacken, Hosen, Mänteln, Masken, Unterwäsche, etc. verwendet werden.

Generell kann die Innenauskleidung 1 für die Herstellung von Sport-, Freizeit- und/oder Berufsbekleidung verwendet werden.

Mit dieser Innenauskleidung 1 können Ausrüstungsgegenstände 2 hergestellt werden, welche aus einer Schicht 3 aus einem Gewebe und/oder Gewirke bzw. Leder mit antimikrobieller Wirkung und einer weiteren Schicht 4 aus einer semipermeablen Membran 5 hergestellt werden. Die Ausrüstungsgegenstände 2 können zudem eine zusätzliche Schicht 6 aus einem Gewebe und/oder Gewirke bzw. Leder umfassen, welche zur Schicht 3 und weiteren Schicht 4 hinzugefügt wird.

Der Ausrüstungsgegenstand 2 kann zudem noch andere Schichten umfassen, welche beispielsweise dem Ausrüstungsgegenstand 2 gewisse andere Eigenschaften verleihen, wie z.B. durch die Verwendung von flammhemmenden Materialien eine gewisse Hitzebeständigkeit oder durch die Verwendung von schnittfesten Materialien eine gewisse Widerstandsfähigkeit gegenüber scharfen Gegenständen, wie Messer und dgl. Die genannten Eigenschaften können durch die Verwendung spezieller Materialien, wie sie aus dem Stand der Technik, um solche Eigenschaften zu erzielen, bekannt sind, ergänzt werden.

Der erfindungsgemäße Ausrüstungsgegenstand 2 dient zur Herstellung von Bekleidung für die oberen Extremitäten, insbesondere Handschuhe, Fäustlinge, und für die unteren Extremitäten, wie z.B. Socken, Schuhe, Stiefel, Hose, Unterwäsche, für den Kopf, wie z.B. Haube, Kappe, Mütze, Maske und für den Rumpf, wie z.B. Jacke, Mantel, etc.

In einer bevorzugten Ausführungsform wird die erfindungsgemäße Innenauskleidung 1 für die Ausstattung eines Handschuhs, insbesondere eines Arbeits- bzw. Sporthandschuhs verwendet, weil hier geruchshemmende, antimikrobielle, temperaturregulierende, antistatische, wasserabweisendende aber luftdurchlässige Eigenschaften am dringendsten benötigt werden. Ein Handschuh bzw. Fäustling mit erfindungsgemäßer Innenauskleidung 1 kann generell als Berufsbekleidung für Militär, Polizei, Gendarmerie, öffentlicher Dienst, Feuerwehr, etc. verwendet werden.

Die den eigenständigen erfinderischen Lösungen zugrunde liegende Aufgabe kann der Beschreibung entnommen werden.

Der Ordnung halber sei abschließend darauf hingewiesen, dass zum besseren Verständnis des Aufbaus der Innenauskleidung diese bzw. deren Bestandteile teilweise unmaßstäblich und/oder vergrößert und/oder verkleinert dargestellt wurden.

## Patentansprüche

1. Innenauskleidung (1) für einen Ausrüstungsgegenstand (2) zum zumindest temporären Bedecken von Körperteilen, insbesondere Kleidungsstücke, Decken, Schlafsäcke, aus mehreren Schichten, wobei zumindest eine Schicht (3) aus einem Gewebe und/oder Gewirke und zumindest eine weitere Schicht (4) aus einer semipermeablen Membran (5), insbesondere aus Polytetrafluorethylen (PTFE), und/oder einem Laminat, gebildet aus Polyvinylchlorid, Polyurethan, Polyetheramid, Polyetheresteramid, Polyolefinkomponenten, Polyesterkomponenten, oder einem Gemisch daraus angeordnet ist, **dadurch gekennzeichnet, dass** die zumindest eine Schicht (3) aus einem Gewebe und/oder Gewirke mit antimikrobieller Wirkung mit einem Metall ausgebildet ist, wobei das Metall in einem Filament, einer Faser und/oder einer Spinnfaser oder -faden integriert ist.

2. Innenauskleidung (1) nach Anspruch 1, **dadurch gekennzeichnet, dass** das Metall aus einer Gruppe umfassend Silber, Gold, Kupfer, Platinium, und/oder einem Gemisch daraus ausgewählt ist.

3. Innenauskleidung (1) nach Anspruch 1 oder 2, **dadurch gekennzeichnet, dass** das Metall, insbesondere Silber, in einem Prozentanteil mit einer oberen Grenze von 80 %, vorzugsweise 35 %, insbesondere 20 %, und einer unteren Grenze von 0,5 %, vorzugsweise 1 %, insbesondere 5 %, in der Schicht (3) aus einem Gewebe und/oder Gewirke mit antimikrobieller Wirkung enthalten ist.

4. Innenauskleidung (1) nach einem der Ansprüche 1 bis 3, **dadurch gekennzeichnet, dass** das Metall partikelförmig ist und die Partikelgröße aus einem Bereich mit einer unteren Grenze von 0,2 µm, vorzugsweise 5 µm, insbesondere 20 µm und einer oberen Grenze von 500 µm, vorzugsweise 250 µm, insbesondere 200 µm, ausgewählt ist.

5. Innenauskleidung (1) nach einem der Ansprüche 1 bis 4, **dadurch gekennzeichnet, dass** das Metall geflockt, geklebt, laminiert, kaschiert, gedampft, gespritzt, getaucht und/oder geflutet, ist.

6. Innenauskleidung (1) nach einem der Anspruch 1 bis 5, **dadurch gekennzeichnet, dass** die zumindest eine weitere Schicht (4) aus semipermeabler Membran (5) benachbart, gegebenenfalls unmittelbar benachbart, der zumindest einen Schicht (3) aus einem Gewebe und/oder Gewirke mit antimikrobieller Wirkung angeordnet ist.

7. Innenauskleidung (1) nach einem der Anspruch 1 bis 6, **dadurch gekennzeichnet, dass** die zumindest eine Schicht (3) aus einem Gewebe und/oder Gewirke mit antimikrobieller Wirkung auf die zumindest eine weitere Schicht (4) aus semipermeabler Membran (5) flächig oder partiell, insbesondere punktförmig oder netzartig aufgebracht, wie geflockt, geklebt, laminiert, kaschiert, gedampft, gespritzt, getaucht und/oder geflutet, ist.

8. Innenauskleidung (1) nach einem der Ansprüche 1 bis 7, **dadurch gekennzeichnet, dass** die weitere Schicht (4) aus semipermeabler Membran (5) zwischen der zumindest einen Schicht (3) aus einem Gewebe und/oder Gewirke mit antimikrobieller Wirkung und einer zusätzlichen Schicht (6) aus einem Gewebe und/oder Gewirke angeordnet ist.

9. Innenauskleidung (1) nach einem der Ansprüche 1 bis 8, **dadurch gekennzeichnet, dass** die zumindest eine weitere Schicht (4) aus semipermeabler Membran (5), die zumindest eine Schicht (3) aus einem Gewebe und/oder Gewirke mit antimikrobieller Wirkung und gegebenenfalls die zusätzliche Schicht (6) aus einem Gewebe und/oder Gewirke miteinander flächig verbunden sind.

10. Innenauskleidung (1) nach einem der Ansprüche 1 bis 9, **dadurch gekennzeichnet, dass** die zumindest eine weitere Schicht (4) aus semipermeabler Membran (5), die zumindest eine Schicht (3) aus einem Gewebe und/oder Gewirke mit antimikrobieller Wirkung und gegebenenfalls die zusätzliche Schicht (6) aus einem Gewebe und/oder Gewirke zumindest über einen Teil der Fläche rutschfest miteinander verbunden sind.

11. Innenauskleidung (1) nach einem der Ansprüche 1 bis 10, **dadurch gekennzeichnet, dass** wenigstens die zumindest eine Schicht (3) aus einem Gewebe und/oder Gewirke mit antimikrobieller Wirkung und die zumindest eine weitere Schicht (4) aus semipermeabler Membran (5) und gegebenenfalls die zusätzliche Schicht (6) aus einem Gewebe und/oder Gewirke zumindest partiell, insbesondere an ihren Kontaktflächen in Form einer Vielzahl von Punkten oder netzartig, gegebenenfalls aktivierbaren Haftschicht, miteinander verbunden sind.

12. Innenauskleidung (1) nach einem der Ansprüche 1 bis 11, **dadurch gekennzeichnet, dass** die Schichten (3, 4, 6) beispielsweise durch kleben, schweißen, heiß siegeln, laminieren und/oder nähen entlang von Verbindungslinien miteinander verbunden sind.

13. Innenauskleidung (1) nach einem der Ansprüche 1 bis 12, **dadurch gekennzeichnet, dass** diese aus wenigstens je einer Schicht (3) von miteinander an Verbindungslinien verbundenen Schnittteilen wenigstens eines Gewebes und/oder Gewirkes mit antimikrobieller Wirkung einerseits und wenigstens einer weiteren Schicht (4) aus semipermeabler Membran (5) andererseits besteht, wobei die einzelnen Schichten (3, 4) rutschfest und semipermeabel über ihre gesamte gemeinsame Fläche miteinander verbunden sind, wobei die Verbindungslinien (7) der weiteren Schicht (4) aus semipermeabler Membran (5) geschweißt sind, wobei die Verbindungslinien (7) der Teile der Schicht (3) aus einem Gewebe und/oder Gewirke mit antimikrobieller Wirkung unabhängig von der weiteren Schicht (4) aus semipermeabler Membran (5) miteinander verbunden, vorzugsweise genäht, sind.

14. Innenauskleidung nach einem der Ansprüche 1 bis 13, mit wenigstens einer Schicht (3) aus einem Gewebe und/oder Gewirke mit antimikrobieller Wirkung und einer zusätzlichen Schicht (6) aus einem Gewebe und/oder Gewirke, wobei die weitere Schicht (4) aus semipermeabler Membran (5) zwischen den beiden Schichten (3, 6) angeordnet ist, wobei alle Schichten (3, 4, 6) rutschfest und semipermeabel über ihre gesamte gemeinsame Fläche miteinander verbunden sind und die weitere Schicht (4) aus semipermeabler Membran (5) gemeinsame Verbindungslinien (7) mit zumindest einer der beiden Schichten (3, 6) aufweist, die geschweißt bzw. heißgesiegelt, und vorzugsweise auch genäht sind.

15. Innenauskleidung nach einem der Ansprüche 1 bis 14, **dadurch gekennzeichnet, dass** ein- oder beidseitig der semipermeabel miteinander verbundenen Schicht(en) (3) aus einem Gewebe und/oder Gewirke mit antimikrobieller Wirkung und der weiteren Schicht (4) aus semipermeabler Membran (5) wenigstens eine zusätzliche Schicht (6) aus einem Gewebe und/oder Gewirke und/oder wenigstens eine weitere Schicht (4) aus semipermeabler Membran (5) angeordnet ist, die im wesentlichen über die ganze Fläche rutschfest und semipermeabel mit den semipermeabel verbundenen Schichten (3, 4) aus Gewebe und/oder Gewirke und aus semipermeabler Membran (5) verbunden ist, wobei die Verbindungslinien (7) der Teile der weiteren semipermeablen Membran (5) geschweißt sind, während die Verbindungslinien (7) der Schichten (3, 6) aus einem Gewebe und/oder Gewirke unabhängig von der ersten und/oder der weiteren Membran miteinander verbunden, vorzugsweise genäht, sind.

16. Innenauskleidung (1) nach einem der Ansprüche 1 bis 15, **dadurch gekennzeichnet, dass** die Schichten (3, 6) sowohl ohne als auch mit antimikrobieller Wirkung zumindest teilweise aus Gewebe und/oder Gewirke aus Natur- und/oder Chemiefasern gebildet sind.

17. Innenauskleidung (1) nach Anspruch 16, **dadurch gekennzeichnet, dass** die Naturfasern aus einer Gruppe umfassend Pflanzenfasern, insbesondere Pflanzenhaare, wie z.B. Baumwolle, Bastfasern, wie z.B. Flachs, Hanf, Jute, Hartfasern, wie z.B. Kokos, Sisal, Tierfasern, insbesondere Wolle und Haare, wie z.B. Schafwolle, Lama, Rosshaar, Seide, wie z.B. echte Seide, Wildseide, Mineralfasern und/oder Leder, ausgewählt sind.

18. Innenauskleidung (1) nach Anspruch 17, **dadurch gekennzeichnet, dass** die Chemiefasern aus natürlichen und/oder synthetischen Polymeren und/oder aus organischen Rohstoffen gebildet sind.

19. Innenauskleidung (1) nach Anspruch 18, **dadurch gekennzeichnet, dass** die natürlichen Polymere pflanzlicher und/oder tierischer Herkunft, wie z.B. Cellulose-, Alginat-, regenerierte Proteinfasern und/oder Elastodien (Gummi) sind.

20. Innenauskleidung (1) nach Anspruch 19, **dadurch gekennzeichnet, dass** die synthetischen Polymere Polymerisat-, wie z.B. Polyacryl, Polychlorid, Polyethylen, Polypropylen, Elastodien, Polykondensat- wie z.B. Polyamid, Polyester, und/oder Polyadditionsfasern, wie z.B. Polyurethan, Elastan, sind.

21. Innenauskleidung (1) nach Anspruch 20, **dadurch gekennzeichnet, dass** die organischen Rohstoffe Glasfasern, Metallfasern und/oder Kohlenstöfffasern sind.

22. Innenauskleidung (1) nach einem der Ansprüche 16 bis 21, **dadurch gekennzeichnet, dass** die Schichten (3, 6) sowohl ohne als auch mit antimikrobieller Wirkung zumindest teilweise aus Gewebe und/oder Gewirke aus einer Gruppe umfassend Aramid- und/oder Paraaramidfasern, wie Kevlar® (Poly(p-phenylen-terephthalamid), Nomex® (Aramid aus m-Phenylendiamin und Isophthalsäure), Twaron®, Technora, Teijinconex, Phenol-Formaldehydfasern, wie Kynol, Polyamid/Polyimidfasern wie Kermel, Polybenzimidazolfasem oder Fasergemischen daraus, gebildet sind.

23. Innenauskleidung (1) nach einem der Ansprüche 1 bis 22, **dadurch gekennzeichnet, dass** mehrere Schichten (3) aus einem Gewebe und/oder Gewirke mit antimikrobieller Wirkung angeordnet sind.

24. Innenauskleidung (1) nach einem der Ansprüche 1 bis 23, **dadurch gekennzeichnet, dass** mehrere Schichten (6) aus einem Gewebe und/oder Gewirke bzw. Leder angeordnet sind.

25. Verfahren zur Herstellung einer Innenauskleidung (1) mit welchem zumindest eine Schicht (3) aus einem Gewebe und/oder Gewirke mit antimikrobieller Wirkung mit einem Metall, wobei das Metall in einem Filament, einer Faser und/oder einer Spinnfaser oder - faden integriert ist und eine weitere Schicht (4) aus semipermeabler Membran (5) der gewünschten Innenauskleidung (1) entsprechend in einzelne Schnittteile zugeschnitten und geformt werden, worauf die zumindest zwei Schichten (3, 4) zumindest partiell deckend, und gegebenenfalls semipermeabel, miteinander verbunden werden.

26. Verfahren zur Herstellung einer Innenauskleidung (1) nach Anspruch 25, **dadurch gekennzeichnet, dass** mehrere Schichten, insbesondere zumindest eine Schicht (3) aus einem Gewebe und/oder Gewirke mit antimikrobieller Wirkung, eine weitere Schicht (4) aus semipermeabler Membran (5) und eine zusätzliche Schicht (6) aus einem Gewebe und/oder Gewirke der gewünschten Innenauskleidung (1) entsprechend in einzelne Schnittteile zugeschnitten und geformt werden, worauf die Schichten (3, 4, 6) zumindest partiell deckend, und gegebenenfalls semipermeabel, miteinander verbunden werden.

27. Verfahren zur Herstellung einer Innenauskleidung (1), **dadurch gekennzeichnet, dass** die zumindest eine Schicht (3) aus einem Gewebe und/oder Gewirke mit antimikrobieller Wirkung mit einem Metall, wobei das Metall in einem Filament, einer Faser und/oder einer Spinnfaser oder -faden integriert ist und die zumindest eine Schicht (4) aus semipermeabler Membran (5) vor dem Zuschneiden zumindest partiell miteinander verbunden werden, worauf diese Schichten (3, 4) gemeinsam zugeschnitten und die Schnittteile der Innenauskleidungsform entsprechend zusammengefügt werden.

28. Verfahren nach Anspruch 27, **dadurch gekennzeichnet, dass** zumindest eine zusätzliche Schicht (6) aus einem Gewebe und/oder Gewirke bzw. Leder, die zumindest eine Schicht (3) aus einem Gewebe und/oder Gewirke mit antimikrobieller Wirkung und die Schicht (4) aus semipermeabler Membran (5) vor dem Zuschneiden zumindest partiell miteinander verbunden werden, worauf diese Schichten gemeinsam zugeschnitten und die Schnittteile der Innenauskleidungsform entsprechend zusammengefügt werden.

29. Verfahren nach einem der Ansprüche 25 bis 28, **dadurch gekennzeichnet, dass** die Schicht (3) aus einem Gewebe und/oder Gewirke mit antimikrobieller Wirkung gegengleich zur Schicht (6) aus einem Gewebe und/oder Gewirke zugeschnitten wird.

30. Verfahren nach einem der Ansprüche 25 bis 29, **dadurch gekennzeichnet, dass** die Schicht (3) aus einem Gewebe und/oder Gewirke mit antimikrobieller Wirkung gegengleich zu der Schicht (4) aus semipermeabler Membran (5) und der zusätzlichen Schicht (6) aus einem Gewebe und/oder Gewirke bzw. Leder zugeschnitten wird.

31. Verwendung der Innenauskleidung (1) nach einem der Ansprüche 1 bis 24, für Handschuhe, Fäustlinge, Mütze, Haube, Kappe, Socken, Schuhe, Stiefel, Jacke, Hose, Mantel, Maske, Unterwäsche etc.

32. Verwendung der Innenauskleidung (1) nach einem der Ansprüche 1 bis 24 für Sport-, Freizeit- und/oder Berufsbekleidung.

33. Ausrüstungsgegenstand (2) mit einer Innenauskleidung (1) nach einem der Ansprüche 1 bis 24, **dadurch gekennzeichnet, dass** die Innenauskleidung (1) aus einer Schicht (4) aus semipermeabler Membran (5) und einer Schicht (3) aus Gewebe und/oder Gewirke mit antimikrobiellen Eigenschaften und gegebenenfalls einer zusätzlichen Schicht (6) aus einem Gewebe und/oder Gewirke bzw. Leder gebildet ist.

34. Ausrüstungsgegenstand (2) nach Anspruch 33, **dadurch gekennzeichnet, dass** dieser als Bekleidung für die oberen Extremitäten, insbesondere Handschuhe, für die unteren Extremitäten, wie z.B. Socken, Schuhe, Stiefel, Hosen, Unterwäsche, für den Kopf, wie z.B. Haube, Kappe, Mütze, Maske, und für den Rumpf, wie z.B. Jacke, Mantel, verwendet wird.

## Claims

1. Internal lining (1) for an item of equipment (2) for at least temporarily covering body parts, in particular items of clothing, quilts, sleeping bags, comprising several layers, and at least one layer (3) made from a woven and/or non-woven fabric and at least one other layer (4) of a semi-permeable membrane (5) made from polytetrafluoroethylene (PTFE), and/or a laminate comprising polyvinyl chloride, polyurethane, polyether amide, polyether ester amide, polyolefin components, polyester components or a mixture of these are provided, **characterised in that** the at least one layer (3) with an anti-microbial effect made from a woven and/or non-woven fabric contains a metal, which metal is integrated in a filament, a fibre and/or a spunbonded fibre or spunbonded yarn.

2. Internal lining (1) as claimed in claim 1, **characterised in that** the metal is selected from a group comprising silver, gold, copper, platinum and/or a mixture of these.

3. Internal lining (1) as claimed in claim 1 or 2, **characterised in that** the metal, in particular silver, represents a proportion by percentage with an upper threshold of 80 %, preferably 35 %, in particular 20 %, and a lower threshold of 0.5 %, preferably 1 %, in particular 5 %, of the layer (3) made from a woven and/or non-woven fabric or leather with an anti-microbial effect.

4. Internal lining (1) as claimed in one of claims 1 to 3, **characterised in that** the metal is in particulate form and the particle size is selected from a range with a lower threshold of 0.2 µm, preferably 5 µm, in particular 20 µm and an upper threshold of 500 µm, preferably 250 µm, in particular 200 µm.

5. Internal lining (1) as claimed in one of claims 1 to 4, **characterised in that** the metal is flocculated, adhered, laminated, coated, vapour deposited, sprayed, dipped and/or impregnated.

6. Internal lining (1) as claimed in one of claims 1 to 5, **characterised in that** the at least one other layer (4) of semi-permeable membrane (5) is disposed adjacent, optionally directly adjacent, to the at least one layer (3) made from a woven fabric and/ or knitted fabric with an anti-microbial effect.

7. Internal lining (1) as claimed in one of claims 1 to 6, **characterised in that** the at least one layer (3) made from a woven and/or non-woven fabric with an anti-microbial effect is applied to the at least one other layer (4) of semi-permeable membrane (5) by its full surface or partially, in particular in a dotted or network pattern, such as flocculated, adhered, laminated, coated, vapour deposited, coated, sprayed, dipped and/or impregnated.

8. Internal lining (1) as claimed in one of claims 1 to 7, **characterised in that** the other layer (4) of semi-permeable membrane (5) is disposed between the at least one layer (3) made from a woven and/or non-woven fabric with an anti-microbial effect and an additional layer (6) made from a woven and/or non-woven fabric.

9. Internal lining (1) as claimed in one of claims 1 to 8, **characterised in that** the at least one other layer (4) of semi-permeable membrane (5), the at least layer (3) made from a woven and/or non-woven fabric with an anti-microbial effect and optionally the additional layer (6) made from a woven and/or non-woven fabric are joined to one another by their surface.

10. Internal lining (1) as claimed in one of claims 1 to 9, **characterised in that** the at least one other layer (4) of semi-permeable membrane (5), the at least one layer (3) made from a woven and/or non-woven fabric with an anti-microbial effect and optionally the additional layer (6) made from a woven and/or non-woven fabric are joined to one another across a part of the surface in a non-slip arrangement.

11. Internal lining (1) as claimed in one of claims 1 to 10, **characterised in that** at least the at least one layer (3) made from a woven and/or non-woven fabric with an anti-microbial effect and the at least one other layer (4) of semi-permeable membrane (5) and optionally the additional layer (6) made from a woven and/or non-woven fabric are joined to one another at least partially, in particular at their contact surfaces, in the form of a plurality of dots or a network pattern, optionally by an activatable adhesive layer.

12. Internal lining (1) as claimed in one of claims 1 to 11, **characterised in that** the layers (3, 4, 6) are joined to one another by bonding, welding, hot sealing, lamination and/or stitching along join lines, for example.

13. Internal lining (1) as claimed in one of claims 1 to 12, **characterised in that** it comprises at least one layer (3) respectively of pattern pieces of at least a woven and/or non-woven fabric with an anti-microbial effect on the one hand and at least one other layer (4) of semi-permeable membrane (5) on the other hand joined to one another along join lines, and the individual layers (3, 4) are joined to one another so as to be non-slip and semi-permeable across their entire joint surface, and the join lines (7) of the other layer (4) of semipermeable membrane (5) are welded, and the join lines (7) of the parts of the layer (3) made from a woven and/or non-woven fabric with an anti-microbial effect are joined to one another separately from the other layer (4) of semi-permeable membrane (5), preferably stitched.

14. Internal lining as claimed in one of claims 1 to 13, with at least one layer (3) made from a woven and/or non-woven fabric with an anti-microbial effect and an additional layer (6) made from a woven and/or non-woven fabric, and the other layer (4) of semi-permeable membrane (5) is disposed between the two layers (3, 6), and all the layers (3, 4, 6) are joined to one another across their entire common surface so as to be non-slip and semipermeable and the other layer (4) of semi-permeable membrane (5) has common join lines (7) with at least one of the two layers (3, 6), which are welded or heat sealed, and preferably also stitched.

15. Internal lining as claimed in one of claims 1 to 14, **characterised in that** on one or both sides, the mutually joined semi-permeable layer(s) (3) made from a woven and/or non-woven fabric with an anti-microbial effect and the other layer (4) of semi-permeable membrane (5) is provided with at least one additional layer (6) made from a woven and/or non-woven fabric and/or at least one other layer (4) of semi-permeable membrane (5), which is joined to the semi-permeable joined layers (3, 4) made from woven and/or non-woven fabric and of semi-permeable membrane (5) essentially across the entire surface so as to be non-slip and semi-permeable, and the join lines (7) of the parts of the other semi-permeable membrane (5) are welded, whilst the join lines (7) of the layers (3, 6) made from a woven and/or non-woven fabric are joined to one another separately from the first and/or the other membrane, preferably stitched.

16. Internal lining (1) as claimed in one of claims 1 to 15, **characterised in that** the layers (3, 6) both without and with an anti-microbial effect made from woven and/or non-woven fabric are at least partially made from natural and/or chemical fibres.

17. Internal lining (1) as claimed in claim 16, **characterised in that** the natural fibres are selected from a group comprising vegetable fibres, in particular plant fibres such as cotton for example, bast fibres such as flax, hemp, jute for example, hard fibres such as coconut, sisal for example, animal fibres, in particular wool and hair such as sheepswool, llama, horse hair, for example, silk, such as real silk, wild silk for example, mineral fibres and/or leather.

18. Internal lining (1) as claimed in claim 17, **characterised in that** the chemical fibres are natural and/or synthetic polymers and/or organic raw materials.

19. Internal lining (1) as claimed in claim 18, **characterised in that** the natural polymers are of vegetable and/or animal origin, such as cellulose fibres, alginate fibres, regenerated protein fibres and/or elasto-diene (rubber).

20. Internal lining (1) as claimed in claim 19, **characterised in that** the synthetic polymers are polymerisate fibres, such as polyacrylic, polychloride, polyethylene, polypropylene fibres for example, elasto-diene, polycondensate fibres such as polyamide, polyester, and/or polyaddition fibres such as polyurethane, elastane for example.

21. Internal lining (1) as claimed in claim 20, **characterised in that** the organic raw materials are glass fibres, metal fibres and/or carbon fibres.

22. Internal lining (1) as claimed in one of claims 16 to 21, **characterised in that** the layers (3, 6) both without and with an anti-microbial effect are at least partially made from woven and/or non-woven fabric selected from a group comprising aramide and/or para-aramide fibres such as Kevlar® (poly(p-phenylene terephthalamide), Nomex® (aramide comprising m-phenylene diamine and isophthalic acid), Twaron®, Technora, Teijinconex, phenol formaldehyde fibres such as Kynol, polyamide/polyimide fibres such as Kermel, polybenzimidazole fibres or fibre mixtures thereof.

23. Internal lining (1) as claimed in one of claims 1 to 22, **characterised in that** several layers (3) made from a woven and/or non-woven fabric with an anti-microbial effect are provided.

24. Internal lining (1) as claimed in one of claims 1 to 23, **characterised in that** several layers (6) made from a woven and/or non-woven fabric or leather are provided.

25. Method of producing an internal lining (1) whereby at least one layer (3) made from a woven and/or non-woven fabric with an anti-microbial effect containing a metal, which metal is integrated in a filament, a fibre and/or a spunbonded fibre or spunbonded yarn, and another layer (4) of semi-permeable membrane (5), are cut into individual pattern pieces corresponding to the desired internal lining (1) and shaped, after which the at least two layers (3, 4) are joined to one another at least partially congruently and optionally so as to be semi-permeable.

26. Method of producing an internal lining (1) as claimed in claim 25, **characterised in that** several layers, in particular at least one layer (3) made from a woven and/or non-woven fabric with an anti-microbial effect, one other layer (4) of semi-permeable membrane (5) and an additional layer (6) made from a woven and/or non-woven fabric, are cut into individual pattern pieces corresponding to the desired internal lining (1) and shaped, after which the layers (3, 4, 6) are joined to one another at least partially congruently and optionally so as to be semi-permeable.

27. Method of producing an internal lining (1), **characterised in that** the at least one layer (3) made from a woven and/or non-woven fabric with an anti-microbial effect containing a metal, which metal is integrated in a filament, a fibre and/or a spunbonded fibre or spunbonded yarn, and the at least one layer (4) of semi-permeable membrane (5) are cut are at least partially joined to one another prior to cutting, after which these layers (3, 4) are cut jointly and the pattern pieces joined to assume the internal lining shape accordingly.

28. Method as claimed in claim 27, **characterised in that** at least one additional layer (6) made from a woven and/or non-woven fabric or from leather, the at least one layer (3) made from a woven and/or non-woven fabric with an anti-microbial effect and the layer (4) of semi-permeable membrane (5) are at least partially joined to one another prior to cutting, after which these layers are cut jointly and the pattern pieces are joined to assume the internal lining shape accordingly.

29. Method as claimed in one of claims 25 to 28, **characterised in that** the layer (3) made from a woven and/or non-woven fabric with an anti-microbial effect is cut so that it complements the layer (6) made from a woven and/or non-woven fabric or leather.

30. Method as claimed in one of claims 25 to 29, **characterised in that** the layer (3) made from a woven and/or non-woven fabric with an anti-microbial effect is cut so that it complements the layer (4) of semi-permeable membrane (5) and the additional layer (6) made from a woven and/or non-woven fabric or leather.

31. Use of the internal lining (1) as claimed in one of claims 1 to 24 for gloves, mittens, hats, hoods, caps, socks, shoes, boots, jackets, coats, masks, underwear, etc..

32. Use of the internal lining (1) as claimed in one of claims 1 to 24 for sports, leisure and/or professional clothing.

33. Item of equipment (2) with an internal lining (1) as claimed in one of claims 1 to 24, **characterised in that** the internal lining (1) comprises a layer (4) of semi-permeable membrane (5) and a layer (3) made from woven and/or non-woven fabric with anti-microbial properties and optionally an additional layer (6) made from a woven and/or non-woven fabric or leather.

34. Item of equipment (2) as claimed in claim 33, **characterised in that** it is used in an item of clothing for the upper extremities, in particular gloves, for the lower extremities, such as for socks, shoes, boots, trousers, underwear for example, for the head, such as hoods, caps, hats, masks for example, and for the torso such as jackets, coats, etc..

## Revendications

1. Revêtement intérieur (1) pour un objet d'équipement (2) pour couvrir au moins temporairement des parties de corps, en particulier des vêtements, couvertures, sacs de couchage, en plusieurs couches, où au moins une couche (3) en un tissu et/ou tricot et au moins une autre couche (4) en une membrane semi-perméable (5), en particulier en polytétrafluoroéthylène (PTFE), et/ou un laminé, formé en chlorure de polyvinyle, polyuréthane, polyétheramide, polyétheresteramide, composants de polyoléfine, composants de polyester ou d'un mélange de ceux-ci, **caractérisé en ce qu'**au moins une couche précitée (3) est formée par un tissu et/ou tricot avec un effet anti-microbien avec un métal, où le métal est intégré dans un filament, une fibre et/ou une fibre ou fil textile.

2. Revêtement intérieur (1) selon la revendication 1, **caractérisé en ce que** le métal est sélectionné dans un groupe comprenant l'argent, l'or, le cuivre, le platine et/ou un mélange de ceux-ci.

3. Revêtement intérieur (1) selon la revendication 1 ou 2, **caractérisée en ce que** le métal, en particulier l'argent, est contenu en une part en pour cent avec une limite supérieure de 80%, de préférence 35%, en particulier 20%, et une limite inférieure de 0,5%, de préférence 1%, en particulier 5% dans la couche (3) en un tissu et/ou tricot avec effet anti-microbien.

4. Revêtement intérieur (1) selon l'une des revendications 1 à 3, **caractérisé en ce que** le métal est en forme de particules, et que la grandeur des particules a été sélectionnée dans une plage avec une limite inférieure de 0,2 µm, de préférence 5µm, en particulier 20 µm et une limite supérieure de 500 µm, de préférence 250 µm, en particulier 200 µm.

5. Revêtement intérieur (1) selon l'une des revendications 1 à 4, **caractérisé en ce que** le métal est floconné, collé, laminé, contre-collé, vaporisé, projeté, plongé et/ou noyé.

6. Revêtement intérieur (1) selon l'une des revendications 1 à 5, **caractérisé en ce qu'**au moins une autre couche précitée (4) en membrane semi-perméable (5) est disposée d'une manière avoisinante, le cas échéant directement avoisinante, à au moins une couche (3) en un tissu et/ou tricot avec un effet anti-microbien.

7. Revêtement intérieur (1) selon l'une des revendications 1 à 6, **caractérisé en ce qu'**au moins une couche (3) en un tissu et/ou tricot avec effet anti-microbien est appliquée sur au moins une autre couche (4) en membrane semi-perméable (5) sur toute la face ou partiellement, en particulier en forme de points où à la manière d'un filet, par exemple floconnée, collée, laminée, contre-collée, vaporisée, projetée, plongée et/ou noyée.

8. Revêtement intérieur (1) selon l'une des revendications 1 à 7, **caractérisé en ce que** l'autre couche (4) en membrane semi-perméable (5) est disposée entre au moins une couche (3) en un tissu et/ou tricot avec effet anti-microbien et une couche additionnelle (6) en un tissu et/ou tricot.

9. Revêtement intérieur (1) selon l'une des revendications 1 à 8, **caractérisé en ce qu'**au moins une autre couche (4) en membrane semi-perméable (5), au moins une couche (3) en un tissu et/ou tricot avec effet anti-microbien et le cas échéant la couche additionnelle (6) en un tissu et/ou tricot sont reliées d'une manière plane les unes aux autres.

10. Revêtement intérieur (1) selon l'une des revendications 1 à 9, **caractérisé en ce qu'**au moins une autre couche (4) en membrane semi-perméable (5), au moins une couche (3) en un tissu et/ou tricot avec effet anti-microbien et le cas échéant la couche additionnelle (6) en un tissu et/ou tricot sont reliées les unes aux autres d'une manière non-glissante sur au moins une partie de la face.

11. Revêtement intérieur (1) selon l'une des revendications 1 à 10, **caractérisé en ce qu'**au moins une couche (3) en un tissu et/ou tricot avec effet anti-microbien et au moins une autre couche (4) en membrane semi-perméable (5) et le cas échéant la couche additionnelle (6) en un tissu et/ou tricot sont reliées les unes aux autres au moins partiellement, en particulier à leurs faces de contact sous la forme d'une multitude de points où à la manière d'un filet, le cas échéant par une couche d'adhésion apte à être activée.

12. Revêtement intérieur (1) selon l'une des revendications 1 à 11, **caractérisé en ce que** les couches (3,4,6) sont reliées entre elles par exemple par collage, soudage, scellement à chaud, laminage et/ou couture le long de lignes de liaison.

13. Revêtement intérieur (1) selon l'une des revendications 1 à 12, **caractérisé en ce que** celui-ci est constitué d'au moins respectivement une couche (3) de parties de coupe reliées à des lignes de liaison d'au moins un tissu et/ou tricot avec effet anti-microbien, d'une part, et d'au moins une autre couche (4) en membrane semi-perméable (5), d'autre part, où les couches individuelles (3,4) sont reliées les unes aux autres d'une manière non-glissante et de manière semi-perméable sur toute leur face commune, où les lignes de liaison (7) de l'autre couche (4) en membrane semi-perméable (5) sont soudées, où les lignes de liaison (7) des parties de la couche (3) en un tissu et/ou tricot avec effet anti-microbien sont reliées les unes aux autres, de préférence par couture, indépendamment de l'autre couche (4) en membrane semi-perméable (5).

14. Revêtement intérieur selon l'une des revendications 1 à 13, avec au moins une couche (3) en un tissu et/ou tricot avec effet anti-microbien et une couche additionnelle (6) en un tissu et/ou tricot, où l'autre couche (4) en membrane semi-perméable (5) est disposée entre les deux couches (3, 6), où toutes les couches (3,4,6) sont reliées les unes aux autres d'une manière non-glissante et semi-perméable sur toute leur face commune et l'autre couche (4) en membrane semi-perméable (5) présente des lignes de liaison communes (7) avec au moins une des deux couches (3, 6), qui sont soudées respectivement scellées à chaud et de préférence également cousues.

15. Revêtement intérieur selon l'une des revendications 1 à 14, **caractérisé en ce qu'**il est disposé sur un ou les deux côtés de la ou des couches (3) reliées d'une manière semi-perméable les unes aux autres en un tissu et/ou tricot avec effet anti-microbien et l'autre couche (4) en membrane semi-perméable (5) au moins une couche additionnelle (6) en un tissu et/ou tricot et/ou au moins une autre couche (4) en membrane semi-perméable (5), qui est reliée sensiblement sur toute la face d'une manière non-glissante et semi-perméable avec les couches (3,4) reliées de manière semi-perméable en tissu et/ou tricot et en membrane semi-perméable (5), où les lignes de liaison (7) des parties de l'autre membrane semi-perméable (5) sont soudées, tandis que les lignes de liaison (7) des couches (3,6) en un tissu et/ou tricot sont reliées, de préférence cousues, entre elles indépendamment de la première et/ou membrane ultérieure.

16. Revêtement intérieur (1) selon l'une des revendications 1 à 15, **caractérisé en ce que** les couches (3,6) à la fois sans et aussi avec effet anti-microbien sont formées au moins partiellement en tissu et/ou tricot en fibres naturelles et/ou chimiques.

17. Revêtement intérieur (1) selon la revendication 16, **caractérisé en ce que** les fibres naturelles sont sélectionnées dans un groupe comprenant des fibres de plantes, en particulier des poils de plantes, comme par exemple le coton, des fibres de filasse, comme par exemple le lin, le chanvre, le jut, des fibres dures, comme par exemple le coco, le sisal, des fibres d'animaux, en particulier de la laine et des poils, comme par exemple, la laine de mouton, de lama, le crin de cheval, la soie, comme par exemple, la soie écrue, soie sauvage, des fibres minéraux et/ou du cuir.

18. Revêtement intérieur (1) selon la revendication 17, **caractérisé en ce que** les fibres chimiques sont formées par des polymères naturels et/ou synthétiques et/ou des matières premières organiques.

19. Revêtement intérieur (1) selon la revendication 18, **caractérisé en ce que** les polymères naturels ont une provenance végétale et/ou animale, comme par exemple, des fibres de cellulose, d'alignate, de protéines régénérées et/ou d'élastodiènes (caoutchouc).

20. Revêtement intérieur (1) selon la revendication 19, **caractérisé en ce que** les polymères synthétiques sont un produit de polymérisation, comme par exemple, polyacryle, polychlorure, polyéthylène, polypropylène, élastodiène, un polycondensat, comme par exemple, le polyamide, polyester, et/ou des fibres de polyaddition, comme par exemple, le polyuréthane, l'élastane.

21. Revêtement intérieur (1) selon la revendication 20, **caractérisé en ce que** les matières premières organiques sont des fibres de verre, des fibres métalliques et/ou des fibres de carbone.

22. Revêtement intérieur (1) selon l'une des revendications 16 à 21, **caractérisé en ce que** les couches (3,6), à la fois sans et aussi avec effet anti-microbien sont formées au moins partiellement d'un tissu et/ou tricot d'un groupe comprenant des fibres aramide et/ou para-aramide, comme Kevlar® (poly(p-phenylen-terephthalamide), Nomex® (aramid à partir de m-phenyl-endiamine et acide isophthalique), Twaron®, Technora, Teijinconex, fibres de phénol-formaldéhyde, comme kynol, fibres de polyamide/polyimide comme kermel, fibres de polybenzimidazole ou leurs mélanges de fibres.

23. Revêtement intérieur (1) selon l'une des revendications 1 à 22, **caractérisé en ce que** plusieurs couches (3) en un tissu et/ou tricot avec effet anti-microbien sont disposées.

24. Revêtement intérieur (1) selon l'une des revendications 1 à 23, **caractérisé en ce que** plusieurs couches (6) en un tissu et/ou tricot respectivement cuir sont disposées.

25. Procédé de fabrication d'un revêtement intérieur (1) par lequel sont découpées et formées en des parties de coupe individuelles au moins une couche (3) en un tissu et/ou tricot avec effet anti-microbien avec un métal, où le métal est intégré dans un filament, une fibre et/ou une fibre ou fil textile, et une autre couche (4) en membrane semi-perméable (5) d'une manière correspondante au revêtement intérieur souhaité (1), suite à quoi au moins deux couches précitées (3,4), avec un recouvrement au moins partiel, et le cas échéant d'une manière semi-perméable, sont reliées l'une à l'autre.

26. Procédé de fabrication d'un revêtement intérieur (1) selon la revendication 25, **caractérisé en ce que** plusieurs couches, en particulier au moins une couche (3) en un tissu et/ou tricot avec effet anti-microbien, une autre couche (4) en membrane semi-perméable (5) et une couche additionnelle (6) en un tissu et/ou tricot, conformément au revêtement intérieur souhaité (1), sont découpées et formées en parties découpées individuelles, suite à quoi les couches (3,4,6), avec un recouvrement au moins partiel et le cas échéant de manière semi-perméable, sont reliées entre elles.

27. Procédé de fabrication d'un revêtement intérieur (1), **caractérisé en ce qu'**au moins une couche (3) en un tissu et/ou tricot avec effet anti-microbien avec un métal, où le métal est intégré dans un filament, une fibre et/ou une fibre ou fil textile et au moins une couche (4) en membrane semi-perméable (5), avant la découpe, sont reliées entre elles au moins partiellement, suite à quoi ces couches (3,4) sont découpées en commun et les parties découpées sont assemblées d'une manière correspondante à la forme du revêtement intérieur.

28. Procédé selon la revendication 27, **caractérisé en ce qu'**au moins une couche additionnelle (6) en un tissu et/ou tricot respectivement en cuir, au moins une couche (3) en un tissu et/ou tricot avec effet anti-microbien et la couche (4) en membrane semi-perméable (5), avant la découpe, sont reliées au moins partiellement entre elles, suite à quoi ces couches sont découpées en commun et les parties découpées sont assemblées d'une manière correspondante à la forme du revêtement intérieur.

29. Procédé selon l'une des revendications 25 à 28, **caractérisé en ce que** la couche (3) en un tissu et/ou tricot avec effet anti-microbien est découpée d'une manière diamétralement opposée à la couche (6) en un tissu et/ou tricot.

30. Procédé selon l'une des revendications 25 à 29, **caractérisé en ce que** la couche (3) en un tissu et/ou tricot avec effet anti-microbien est découpée d'une manière diamétralement opposée à la couche (4) en membrane semi-perméable (5) et la couche additionnelle (6) en un tissu et/ou tricot, respectivement cuir.

31. Utilisation du revêtement intérieur (1) selon l'une des revendications 1 à 24, pour des gants, moufles, casquettes, bonnets, capuchons, chaussettes, chaussures, bottes, vestes, pantalons, manteaux, masques, sous-vêtements etc.

32. Utilisation du revêtement intérieur (1) selon l'une des revendications 1 à 24, pour des vêtements de sport, de loisir et/ou de travail.

33. Objet d'équipement (2) avec un revêtement intérieur (1) selon l'une des revendications 1 à 24, **caractérisé en ce que** le revêtement intérieur (1) est formé par une couche (4) en membrane semi-perméable (5) et une couche (3) en tissu et/ou tricot avec des effets anti-microbiens et le cas échéant une couche additionnelle (6) en un tissu et/ou tricot respectivement cuir.

34. Objet d'équipement (2) selon la revendication 33, **caractérisé en ce que** celui-ci est utilisé comme vêtement pour les extrémités supérieures, en particulier des gants, pour les extrémités inférieures, comme par exemple des chaussettes, chaussures, bottes, pantalons, sous-vêtements, pour la tête, comme par exemple capuchons, bonnets, casquettes, masques et pour le tronc, comme par exemple vestes, manteaux.
